(19) Europäisches Patentamt
European Patent Office
Office européen des brevets

(11) Veröffentlichungsnummer: **0 227 046**
**A2**

(12) **EUROPÄISCHE PATENTANMELDUNG**

(21) Anmeldenummer: **86117656.8**

(22) Anmeldetag: **18.12.86**

(51) Int. Cl.⁴: **C07D 213/69**, C07D 413/12, A01N 43/40, A01N 43/88

(30) Priorität: **21.12.85 DE 3545569**

(43) Veröffentlichungstag der Anmeldung: **01.07.87 Patentblatt 87/27**

(84) Benannte Vertragsstaaten: **AT BE CH DE ES FR GB GR IT LI NL**

(71) Anmelder: **HOECHST AKTIENGESELLSCHAFT**
**Postfach 80 03 20**
**D-6230 Frankfurt am Main 80(DE)**

(72) Erfinder: **Koch, Volker, Dr.**
**Altkönigstrasse 5**
**D-6233 Kelkheim(DE)**
Erfinder: **Fuss, Andreas, Dr.**
**Lindigstrasse 24**
**D-8757 Karlstein(DE)**
Erfinder: **Bonin, Werner, Dr.**
**Im Schultzehnten 18**
**D-6233 Kelkheim(DE)**
Erfinder: **Knauf, Werner, Dr.**
**Im Kirschgarten 24**
**D-6239 Eppstein/Taunus(DE)**
Erfinder: **Waltersdorfer, Anna, Dr.**
**Rauenthaler Weg 28**
**D-6000 Frankfurt am Main(DE)**

(54) **Pyridin-Derivate, Verfahren zu ihrer Herstellung, sie enthaltene Mittel und ihre Verwendung als Schädlingsbekämpfungsmittel.**

(57) Die Verbindungen der Formel (I)

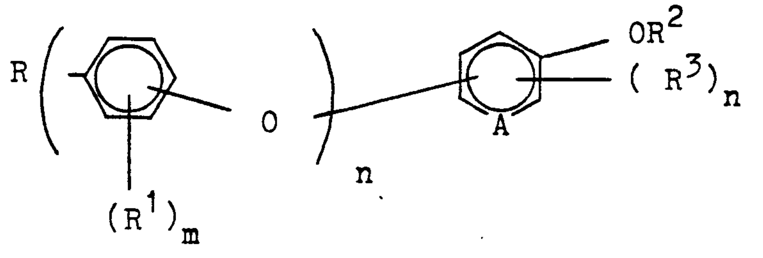 (I),

worin A = N oder N → O,
R = einen Rest der Formeln $R^4$-$CY^1$-$NR^5$-$CY^2$-$NR^6$-,

EP 0 227 046 A2

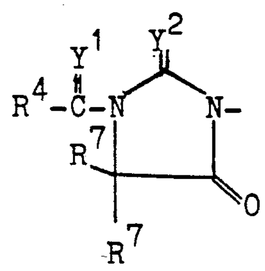
,
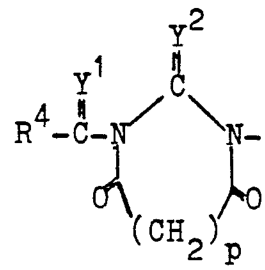
,
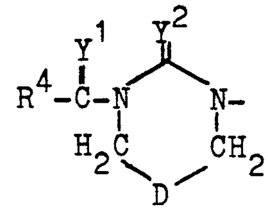
,

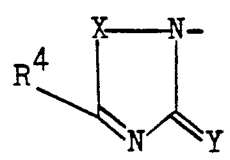
,
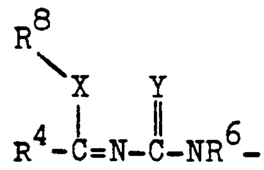
,
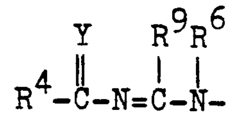

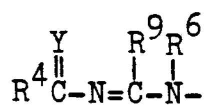

oder

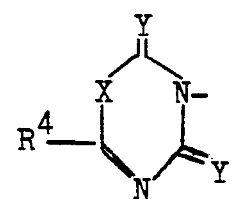
;

$R^1$ Halogen, Alkyl, Alkenyl, Alkinyl, Alkoxy, Alkoxycarbonyl, Alkylcarbonyl, wobei die Reste halogeniert sein können, Ni tro, Cyano oder Carboxy, $R^2$ halogeniertes Alkoxy, halogeniertes Alkenyloxy oder halogeniertes Alkinyloxy, $R^3$ Halogen, n = 0, 1 oder 2, m = 0, 1, 2 oder 3 und p 0 oder 1 bedeuten, sowie deren Salze eignen sich vorteilhaft zur Bekämpfung von Schadinsekten, Akariden, Nematoden oder Mollusken.

**Pyridin-Derivate, Verfahren zu ihrer Herstellung, sie enthaltene Mittel und ihre Verwendung als Schädlingsbekämpfungsmittel**

Pyridin-haltige Imidate, Benzoylharnstoffe und Oxdiazindione zur Bekämpfung von tierischen Schädlingen im Pflanzenschutz sind aus EP-A 109 211, DE-A 2810830 und EP-A 138756 bekannt. Es wurden neue Pyridin-Derivate mit vorteilhaften Wirkungen gegen Pflanzenschädlinge gefunden.

Gegenstand der vorliegenden Erfindung sind daher die Verbindungen der allgemeinen Formel (I)

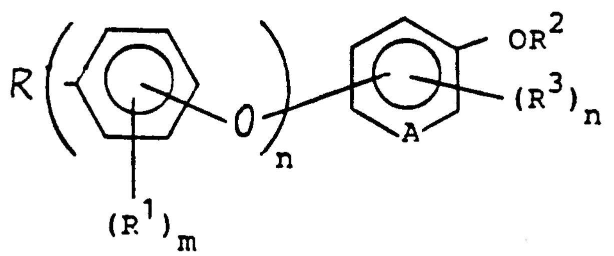 (I),

worin
A = N oder N → O,
R = einen Rest der Formeln

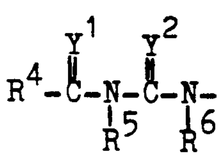 ,

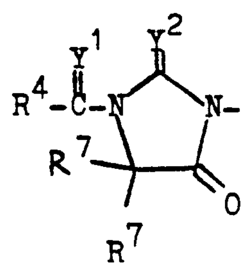 ,

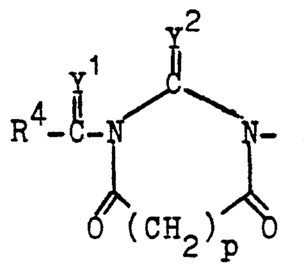, 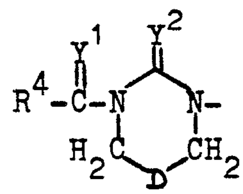,

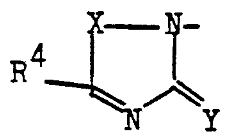, 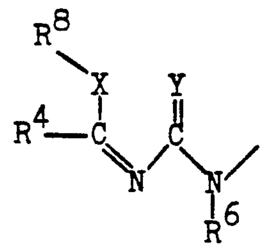,

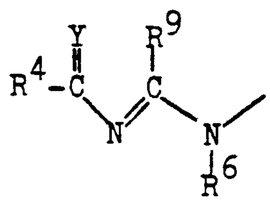, 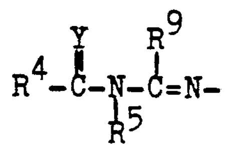 oder 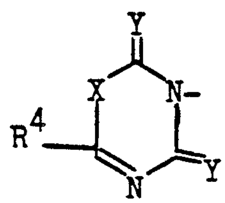,

$R^1$ jeweils unabhängig voneinander Halogen, $(C_1$-$C_6)$-Alkyl, $(C_2$-$C_6)$-Alkenyl, $(C_2$-$C_6)$-Alkinyl, $(C_1$-$C_6)$-Alkoxy, $(C_1$-$C_6)$-Alkoxy-carbonyl, $(C_1$-$C_6$-Alkyl)-carbonyl, wobei die Reste ein-oder mehrfach durch Halogen substituiert sein können, Nitro, Cyano oder Carboxy,

$R^2$ jeweils unabhängig voneinander halogeniertes $(C_1$-$C_6)$-Alkyl, halogeniertes $(C_2$-$C_6)$-Alkenyl oder halogeniertes $(C_2$-$C_6)$-Alkinyl,

$R^3$ unabhängig voneinander Halogen,

$R^4$ Phenyl, das durch Halogen, $(C_1$-$C_3)$-Alkyl, $(C_1$-$C_3)$-Alkoxy, Halo$(C_1$-$C_3)$-alkyl oder Halo$(C_1$-$C_3)$-alkoxy substituiert sein kann,

$R^5$ Wasserstoff, $(C_1$-$C_6)$-Alkoxy, $(C_1$-$C_6)$Alkylthio, Benzyl, halogeniertes $(C_1$-$C_6)$-Alkoxy, halogeniertes $(C_1$-$C_6)$-Alkylthio oder halogeniertes Benzyl,

$R^6$ H, $(C_1$-$C_6)$-Alkyl, $(C_1$-$C_6)$Alkylthio, $(C_1$-$C_6)$-Alkylsulfonyl, die alle halogeniert sein können, Phenylthio oder Phenylsulfonyl, das durch $(C_1$-$C_6)$-Alkyl, $(C_1$-$C_6)$-Alkoxy, $(C_1$-$C_6)$-Alkoxycarbonyl, halogeniertes $(C_1$-$C_6)$-Alkyl, halogeniertes $(C_1$-$C_6)$-Alkoxy, halogeniertes $(C_1$-$C_4)$-Alkoxycarbonyl, Halogen, Nitro oder Cyano substituiert ist, Phosphoryl, Thiophosphoryl, die beide durch zwei Reste der Gruppe $(C_1$-$C_6)$-Alkoxy, $(C_1$-$C_6)$-Alkylthio, Amino, $(C_1$-$C_6)$-Alkylamino oder Di-$(C_1$-$C_6$-Alkyl)-amino substituiert sind,

$R^7$ unabhängig voneinander H, Halogen oder $(C_1$-$C_3)$-Alkyl,

$R^8$ $(C_1$-$C_8)$-Alkyl, $(C_2$-$C_8)$-Alkenyl, $(C_2$-$C_8)$-Alkinyl, Phenyl oder Benzyl, die alle halogeniert sein können,

$R^9$ einen Rest der Formeln $-XR^8$, $-S-S-R^{10}$, Benzimidazolyl, Benztriazolyl, Pyrazolyl, wobei diese drei Reste durch $(C_1$-$C_3)$-Alkyl oder Halogen substituiert sein können, Triazolyl oder Imidazolyl, die beide durch $(C_1$-$C_3)$-Alkyl substituiert sein können,

$R^{10}$ $(C_1$-$C_{18})$-Alkyl, $(C_3$-$C_8)$-Cycloalkyl, Phenyl-$(C_1$-$C_4)$-alkyl oder Phenyl, die alle durch Halogen, Nitro oder $(C_1$-$C_8)$-Alkyl substituiert sein können,

D gegebenenfalls verzweigtes $C_1$-$C_2$-Alkylen, O oder S,

X, Y, $Y^1$, $Y^2$ unabhängig voneinander O oder S,

n unabhängig voneinander 0, 1 oder 2,

m 0, 1, 2 oder 3 und

p 0 oder 1 bedeuten, sowie deren für die Landwirtschaft einsetzbaren Salze.

Die Salzbildung kann beispielsweise im Falle $R^1$ = Carboxy oder $R^5$ = H erfolgen. Als Salze kommen insbesondere Alkali-, Erdalkalimetallsalze, Ammonium-oder durch organische Reste wie Alkyl, Hydroxyalkyl ein-bis vierfach substituierte Ammonium-Salze infrage.

Wenn n = 1 (für den Phenoxy-rest) ist, ist R bevorzugt paraständig zum Phenoxysauerstoff orientiert. Wenn n = 0 ist, ist der Rest R direkt an den Heterocyclus der Formel I gebunden. Der Phenoxyrest bzw. R ist bevorzugt in Nachbarstellung zum Ringatom A orientiert.

Bevorzugt sind die Verbindungen der Formel I, worin

A = N,

R = einen Rest der Formeln

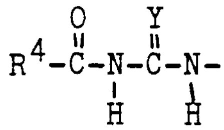 , 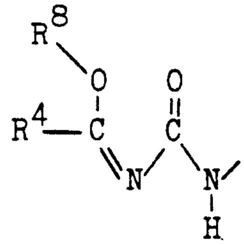 ,

oder 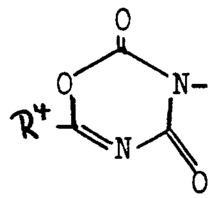

$R^1$ jeweils unabhängig voneinander Halogen, $(C_1-C_6)$-Alkyl oder $(C_1-C_6)$Alkoxy-carbonyl, wobei die Reste ein- oder mehrfach durch Halogen substituiert sein können,

$R^2$ jeweils unabhängig voneinander halogeniertes $(C_1-C_6)$-Alkyl, halogeniertes $(C_2-C_6)$-Alkenyl oder halogeniertes $(C_2-C_6)$-Alkinyl,

$R^3$ unabhängig voneinander Halogen,

$R^4$ Phenyl, das durch Halogen, $(C_1-C_3)$-Alkyl, $(C_1-C_3)$-Alkoxy, Halo$(C_1-C_3)$-alkyl oder Halo$(C_1-C_3)$-alkoxy substituiert sein kann,

$R^8$ $(C_1-C_6)$Alkyl, das halogeniert sein kann,

X unabhängig voneinander O oder S,

n im Falle des Phenoxyrestes 0 oder 1 und im Falle des Restes $R^3$ 0, 1 oder 2 und

m 0, 1, 2 oder 3 bedeuten.

Besonders bevorzugt sind die Verbindungen der Formel I,

worin

A N,

$R^1$ Halogen in den Positionen 2, 3 oder 5 des Phenoxyringes relativ zur Stellung von R, Halo$(C_1-C_6)$alkyl oder $(C_1-C_3)$Alkoxycarbonyl jeweils in den Positionen 3 oder 5 des Phenoxyringes,

$R^2$ $(C_1-C_3)$-Haloalkoxy,

$R^3$ F, Cl oder Br und

$R^4$ Phenyl bedeutet, das durch Halogen, insbesondere in den Positionen 2 oder 6 substituiert ist.

Die Erfindung umfaßt auch alle Stereoisomeren und deren Gemische der Verbindungen der Formel I, wie die E-und Z-Isomere bei ungesättigten Strukturen oder optische Isomere, falls Chiralitätszentren auftreten. Ferner können die Verbindungen der Formel I je nach Substitution in tautomeren Formen vorliegen, die ebenfalls von der Erfindung umfaßt werden.

Gegenstand der vorliegenden Erfindung sind auch Verfahren zur Herstellung der Verbindungen der Formel I, dadurch gekennzeichnet, daß man

a) fürR = $R^4$-$CY^1$-$NR^5$-$CY^2$-$NR^6$-

a1) eine Verbindung der Formel (II) mit

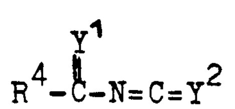 (II)

einer Verbindung der Formel III

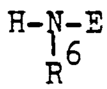 (III), worin E = 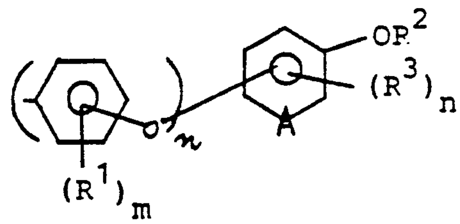

bedeutet, umsetzt, oder

a2) eine Verbindung der Formel IV

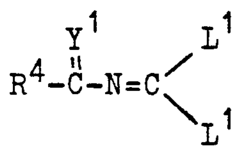 (IV),

worin $L^1$ unabhängig voneinander Halogen, $(C_1\text{-}C_3)$-Alkylthio, $(C_1\text{-}C_3)$-Alkylsulfenyl oder $(C_1\text{-}C_3)$-Alkylsulfonyl bedeutet,

mit einer Verbindung der Formel III und anschließend mit einer Verbindung der Formel $H_2Y$ umsetzt, oder

a3) eine Verbindung der Formel V

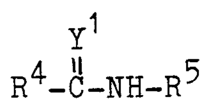 (V),

mit einer Verbindung der Formel VIa

$Y^2=C=N\text{-}E$ (VIa),

oder

a4) eine Verbindung der Formel V mit einer Verbindung der Formel VIb

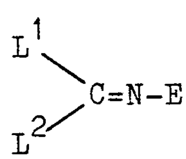 (VIb),

wobei $L^2$ die Bedeutung von $L^1$ besitzt und zusätzlich für Mercapto steht,

und anschließend mit einer Verbindung der Formel $H_2Y$ umsetzt, oder

a5) eine Verbindung der Formel V mit einer Verbindung der Formel VII

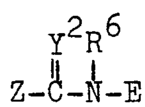 (VII),

worin Z = $(C_1\text{-}C_6)$-Alkoxy, $(C_1\text{-}C_6)$-Alkylthio, Benzyloxy, die alle halogeniert sein können, Triazolyl oder Imidazolyl oder einen Rest der Formel $\text{-NHR}^5$ beeutet, umsetzt, oder

b) für R = 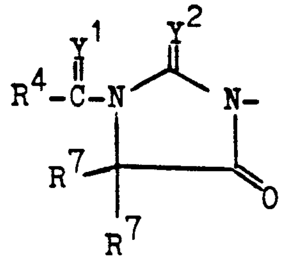

eine Verbindung der Formel VIII

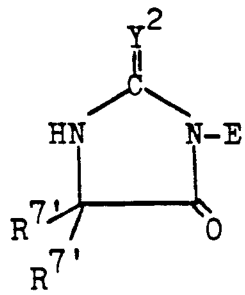 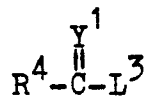

(VIII) (IX),

wobei $R^{7'}$ = H oder ($C_1$-$C_3$)-Alkyl bedeutet, mit einer Verbindung der Formel IX, wobei $L^3$ = eine Abgangsgruppe wie Halogen oder Alkylthio bedeutet, umsetzt und die erhaltenen Verbindungen gegebenfalls halogeniert oder alkyliert, oder

c) für R = 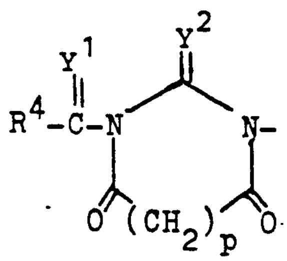 oder 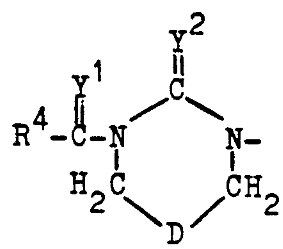

eine gemäß Verfahren a) erhaltene Verbindung der Formel I mit $R^5$, $R^6$ = H mit einer Verbindung Xa oder Xb, worin $L^4$ = eine Abgangsgruppe wie Halogen bedeutet,

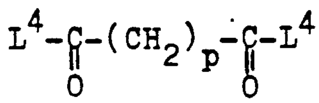

$$L^4-CH_2-D-CH_2-L^4$$

Xa Xb

umsetzt, oder

d) für R = 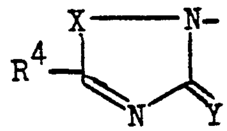

d1) für X = O eine Verbindung der Formel XI mit

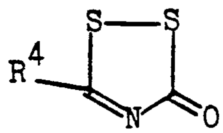 (XI) HONH-E (XII)

einer Verbindung der Formel XII umsetzt und die erhaltene Verbindung der Formel I mit Y = O auf bekannte Weise thioliert, oder

d2) für X = S eine gemäß a) erhaltene Verbindung der Formel I mit Y' = S und $R^5$, $R^6$ = H cyclisiert, oder

e) für R = 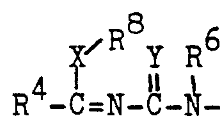

e1) eine Verbindung der Formel XIII

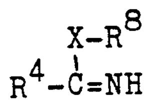

(XIII)

mit einer Verbindung der Formel VIa oder mit einer Verbindung der Formel

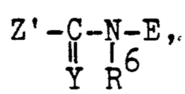

worin Z' die Bedeutung von Z außer $-NHR^5$ besitzt, umsetzt, oder

e2) eine Verbindung der Formel XIV

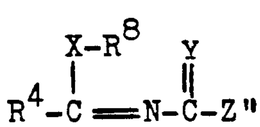 (XIV),

worin Z" die Bedeutung von Z' besitzt oder zusätzlich für Halogen steht, mit einer Verbindung der Formel III umsetzt, oder

f) für R = 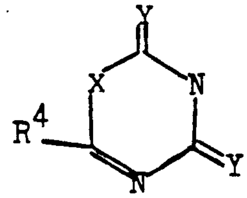

eine Verbindung der Formel II mit einer Verbindung der Formel VIa umsetzt, oder

g) für R = 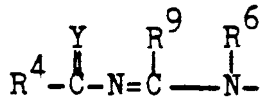

g1) eine Verbindung der Formel XV

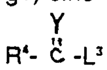 (XV)

mit einer Verbindung der Formel XVI

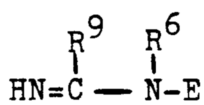 (XVI) umsetzt, oder

g2) eine Verbindung der Formel XVII

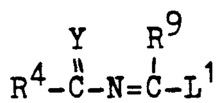 (XVII)

mit einer Verbindung der Formel III umsetzt, oder

g3) für $R^9$ = ein obengenannter Rest außer $-X-R^8$ eine unter a) erhaltene Verbindung der Formel (I) mit $Y^2$ = S und $R^5$, $R^6$ = H mit einer Verbindung der Formel $R^{9'}-SO-R^{9'}$, wobei $R^{9'}$ die Bedeutung von $R^9$ außer $-XR^8$ und $-S-S-R^{10}$ besitzt, oder mit einer Verbindung der Formel $L^5-S-S-R^{10}$, wobei $L^5$ = Halogen, N-Succinyl oder ein analoger Rest bedeutet, umsetzt, oder

h) für R = 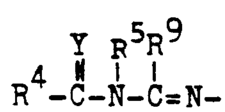

eine Verbindung der Formel XV mit einer Verbindung der Formel XVIII

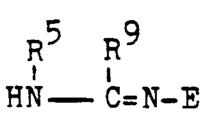 (XVIII) umsetzt.

Die oben unter a) bis h) genannten Verfahrensschritte beruhen auf dem Fachmann bekannten Synthesen.

zu Verfahren a1),a 3) und c) vgl.: DE-OS 2123236
zu Verfahren a2) vgl. : DE-OS 2123236 und E. Kühle et al., Angew. Chem. 79, 671 ff (1967)
zu Verfahren a4) vgl.: Houben-Weyl[1)] Band E 4, S. 1341 ff
zu Verfahren a5) vgl.: Houben-Weyl, Band E 4, S 24 ff
zu Verfahren b) vgl.: EP-A 116103
zu Verfahren d1), d2) vgl.: EP-A 145 095
zu Verfahren e1), e2) vgl.: EP-A 135 894

1) "Houben-Weyl" bedeutet hier wie im folgenden: Houben Weyl, Methoden der organischen Chemie, Georg-Thieme Verlag, Stuttgart.

zu Verfahren f) vgl.: US-PS 4150158
zu Verfahren g1), h) vgl.: Houben-Weyl, Band E 4, S. 593 ff
zu Verfahren g2) vgl.: Houben-Weyl, Band E 4, S. 1018 ff
zu Verfahren g3) vgl.: EP-A 127 245

Die Herstellung der hierzu benötigten Ausgangsverbindungen ist ebenfalls prinzipiell bekannt:
für (II) s. Houben-Weyl, Band E 4, S. 803 ff
für (III) s. Houben-Weyl, Band 11/1, S. 9 ff
für (IV) s. Houben-Weyl, Band E 4, S. 534 ff
für (V) s. Houben-Weyl, Band E 5/1, S. 934 ff, S. 1141 ff
für (VIa), (VIb) s. Deutsche Patentanmeldung P 35 45 570.5 "Neue Pyridin-Derivate und deren N-Oxide, Verfahren zu ihrer Herstellung und ihre Verwendung als Zwischenprodukte (HOE 85/F 295)"
für (VII) s. Houben-Weyl, Band E 4, S. 142 ff

Die Verbindungen (VIII) lassen sich aus den Aminocarbonsäuren der Formel XIX

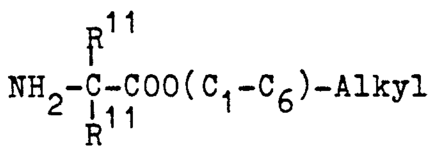 (XIX)

worin $R^{11}$ unabhängig von einander H oder $(C_1-C_3)$-Alkyl bedeutet, durch Umsetzung mit einer Verbindung der Formel VIa (mit Y =O) herstellen, vgl. Chem. Rev. 46, 403 (1950) Houben Weyl, E 5/1 S. 519.

Zur Herstellung von (IX) u. (XV) s. Houben-Weyl, E 5/2, 587 ff
Zur Herstellung von (Xa) s. Houben-Weyl, Band 8, S. 365 ff, 463 ff
Zur Herstellung von (Xb) s. Houben-Weyl, Band 5/3 u. 5/4
Zur Herstellung von (XI) s. Goerdeler, Chem. Ber. 99, S. 782 ff (1966), Chem. Ber. 106, S. 1496 (1973), Heterocycles 5, 189 (1976)
Zur Herstellung von (XII) s. Deutsche Patentanmeldung P 35 45 570.5
Zur Herstellung von (XIII) s. DE-A 3514450
Zur Herstellung von (XIV) s. EP-A 135 894

Die Verbindungen der Formel XVII oder XVIII lassen sich aus den Verbindungen der Formeln XX bzw. XXI durch O-bzw.

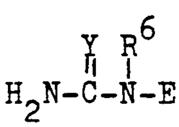 (XX)

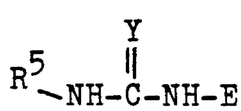 (XXI)

S-Alkylierung herstellen, vgl.
(XX) und XXI werden aus den Verbindungen XXII

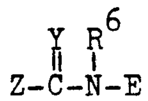 gemäß Houben-Weyl E 4, S. 534 ff

(XXII)

hergestellt. Die Verbindungen der Formel XXII sind aus den Aminoverbindungen der Formel $NH_2$-E, welche in der Deutschen Patentanmeldung P 35 45 570.5 beschrieben sind, durch Phosgenierung, s. Houben-Weyl E 4, S. 738 ff und anschließende Funktionalisierung oder aus den Thioisocyanaten der Formel S = C = N-E auf dem Fachmann geläufigem Wege, s. Houben-Weyl E 4, s. 834 ff oder gemäß anderen bekannten Verfahren zugänglich, s. Houben-Weyl, Band E IV.

Die Verbindungen der Formel XVII werden analog den bekannten Verfahren hergestellt, s. Houben-Weyl E 4, S. 534 ff.

Die Wirkstoffe eignen sich bei guter Pflanzenverträglichkeit und günstiger Warmblütertoxizität zur Bekämpfung von tierischen Schädlingen, insbesondere Insekten, Spinnentieren, Nematoden und Mollusken, ganz besonders bevorzugt zu Bedämpfung von Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den ober erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.,

Aus der Ordnung der Anoplura z.B. Phylloera vastatrix, Pemphigus, spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Bravicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella auroantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Buceulatrix thurberiella, Phyllocnistis citrella, Agrotis spp, Euxoa spp., Feltia spp., Earias insulana, Heliothis spp., Laphygma exigua, Mamestra brassicae, Panolis flammea, Prodenia litura, Spodop tera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariеae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosphila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.
Aus der Ordnung der Arachnida z.B. Scorpio maurus, Latrodectus mactans.

Aus der Klasse der Gastropoda z. B. Deroceras spp., Arion spp., Lymnaea spp., Galba spp., Succinea spp., Biophalaria spp., Bulinus spp., Oncomelania spp.,
Aus der Klasse der Bivalva z. B. Dreissena spp.,

Gegenstand der Erfindung sind auch Mittel, die die Verbindungen der Formal I neben geeigneten Formulierungshilfsmitteln enthalten.

Die erfindungsgemäßen Mittel enthalten die Wirkstoffe der Formel I, im allgemeinen zu 1 -95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungs-oder Inertstoff noch Netzmittel, z.B. polyoxethylierte Alkylphenole, polyoxethylierte Fettalkohole, Alkyl-oder Alkylphenol-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykol ether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise -gewünschtenfalls in Mischung mit Düngemitteln -hergestellt werden.

Die erfindungsgemäßen Wirkstoffe insbesondere die der aufgeführten Beispiele können in ihren handelsüblichen Formulierungen sowie in den aus diesen Formu lierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a.
Bevorzugte Mischungspartner sind

1. aus der Gruppe der Phosphorverbindungen

Acephate, Azamethiphos, Azinphos-ethyl, Azinphosmethyl, Bromophos, Bromophos-ethyl, Chlorfenvinphos, Chlormephos, 1-(4-Clorphenyl-4-(O-ethyl,S-propyl)-phosphoryloxypyrazol (TIA 230), Chlorpyrifos, Chlorpyriphos-methyl, Demeton, Demeton-S-methyl, Demeton-S-methyl sulphone, Dialifos, Diazinon, Dichlorvos, Dicrotophos, Dimethoate, Disulfoton, EPN, Ethion, Ethoprophos, Etrimfos, Famphur, Fenamiphos, Fenitrothion, Fensulfothion, Fenthion, Fonofos, Formothion, Heptenophos, Isazophos, Isofenphos, Isothioate, Isoxathion, Malathion, Mephosfolan, Methacrifos, Methamidophos, Methidathion, Salithion, Mevinphos, Monocrotophos, Naled, Omethoate, Oxydemeton-methyl, Parathion, Parathion-methyl, Phenthoate Phorate, Phosalone, Phosfolan, Phosmet, Phosphamidon, Phoxim, Pirimiphos-ethyl, Pirimiphos-methyl, Profenofos, Propaphos, Propetamphos, Prothiofos, Pyridapenthion, Quinalphos, Sulprofos, Temephos, Terbufos, Tetraclorvinphos, Thiometon, Triazophos, Trichlorphon, Vamidothion;

2. aus der Gruppe der Carbamate

Aldicarb, 2-sec-Butylphenylmethylcarbamate (BPMC), Carbaryl, Carbofuran, Carbosulfan, Cloethocarb, 2,3-Dihydro-2,2-dimethyl-7-benzofuranyl-N-(isopropyl)-N-(carboethoxyethyl)amino)thiomethylcarbamat (OK-174), Ethiofencarb, Furathiocarb, Isoprocarb, Methomyl, 5-Methyl-m-cumenylbutyryl(methyl)carbamate, Oxamyl,

Piromicarb, Propoxur, Thiocarb, Thifanox, Ethyl 4,6,9-triaza-4-benzyl-6,10-dimethyl-8-oxa-7-oxo-5,11-dithia-9-dodecenoate (OK 135), 1-Methylthio-(ethylideneamino) N-methyl-N-(morpholinothio)carbamate (UC 51717);

3. aus der Gruppe der Carbonsäureester

Allethrin, Alphametrin, 5-Benzyl-3-furylmethyl(E)-(1R)-cis-2,2-dimethyl-3-(2-oxothiolan-3-ylidenemethyl)-cyclopropane-carboxylate, Bioallethrin, Bioallethrin((S)-cyclopentylisomer), Bioresmethrin, Biphenate (FMC 54800), α-Cyano-3'-phenoxy-benzyl-2-)4-ethoxyphenyl)-2,2-dichlorocyclopyranecarboxylate (NK 8116, GH 414), (RS)-1-Cyano-1-(6-phenoxy-2-pyridyl)methyl(1RS)-trans-3-(4-tert.butylphenyl)-2,2-dimethylcyclopropanecarboxylate (NCI 85193), Cyfluthrin, Cyhalothrin, Cypermethrin, Deltamethrin, Empenthrin, Esfenvalerate, 5-(4-(4-(4-Ethoxyphenyl)-4-methylpentyl)-2-fluoro-1,3-diphenylether (MTI 800), 3-(2-(4-Ethoxyphenyl-2-methyl-propoxymethyl)-1,3-diphenylether (MTI 500), Fenfluthrin, Fenpropathrin, Fenvalerate,Flucythrinate, Flumethrin, Fluralinate (Disomers), Permethrin, Phenothrin ((R)-isomers), Pyrethrins (natural products), Resmethrin, Tetramethrin, Tralomethrin;

4. aus der Gruppe der Amidine

Amitraz, Chlordimeform;

5. aus der Gruppe der Zinnverbindungen

Cyhexatin, Fenbutatin oxide;

6. Sonstige

Abamectin, Bacillus thuringensis, Bensultap, Binapacryl, Bromopropylate, Buprofezin, Camphechlor, Cartap, Chlorbenzialate, Chlorfluazuron, 2-(4-Chlorphenyl)-4,5-diphenylthiophen (UBI-T 930), Clofentezine, Cyclopropancarbonsäure(2-naphthylmethyl)ester (Ro 12-0470), Cyromazin, DDT, Dicofol, N-(3,5-Dichloro-2,4-difluorphenyl)amino)carbonyl)--2,6-difluorbenzamid (CME 134), N-(3,5-Dichlor-4-(1,1,2,2-tetrafluoroethoxy)phenyl)-amino)carbonyl)-2,6-difluorbenzamide (XRD 473), Diflubenzuron, N-(2,3-Dihydro-3-methyl-1,3-thiazol-2-ylidene)2,4-xylidine, Dinobuton, Dinocap, Endosulfan, Fenoxycarb, Fenthiocarb, Flubenzimine, Gamma-HCH, Hexythiazox, Ivermectin, 2-Nitromethyl-4,5-dihydro-6H-thiazin (SD 52618), 2-Nitromethyl-3,4-dihydrothiazol -(SD 35651), Propargite, Tetradifon, Tetrahydro-5,5-dimethyl-2(1H)-pyrimidinone(3-(4-trifluormethyl)-phenyl)-1-(2-(4-trifluormethyl)phenyl)ethenyl-2-propylidene)hydrazone (AC 217300), Tetrasul, Thiocyclam, Triflumuron, Kernpolyeder-und Granuloseviren.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Ekto-und Endoparasiten beispielsweise von Helminthen oder ektoparasitierenden Insekten auf dem veterinärmedizinischen Gebiet bzw. auf dem Gebiet der Tierhaltung.

Die Anwendung der erfindungsgemäßen Wirkstoffe geschieht hier in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Auf gießens (pour-on and spoton) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck der Insekten abhängig und lassen sich nach den üblichen Methoden leicht ermitteln und festlegen. Die neuen Verbindungen können bei Rindern z.B. in Dosiermengen von 0,01 bis 1 mg/kg Körpergewicht eingesetzt werden.

Nachfolgende Beispiele dienen zur Erläuterung der Erfindung.

## A. Fomulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz-und Dispergiermittel mischt und in einer Stiftmühle mahlt.

c) Ein in Wasser leicht dispergierbares Dispersionskonzentrat stellt man her, indem man 20 Gew.-Teile Wirkstoff mit 6 Gew.-Teilen Alkylphenolpolyglykolether (Triton X 207), 3 Gew.-Teilen Isotridecanolpolyglykolether (8 AeO) und 71 Gew.-Teilen paraffinischem Mineralöl (Siedebereich z.B. ca. 255 bis über 377°C) mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertes Nonylphenol (10 AeO) als Emulgator.

e) Ein Granulat läßt herstellen aus 2 bis 15 Gew.-Teilen Wirkstoff und einem inerten Granulatträgermaterial wie Attapulgit, Bimsgranulat und/oder Quarzsand.

## B. Chemische Beispiele

### Beispiel 1

N-(4-(3-Chlor-5-difluormethoxy-2-pyridyloxy)-3,5-dichlorphenyl)-N'-(2,6-difluorbenzoylharnstoff)

1,27 g (3 mmol) 4-(3-Chlor-5-difluormethoxy-2-pyridyloxy)-3,5-dichloranilin und 0,56 g (3 mmol) frisch destilliertes 2,6-Difluorbenzoylisocyanat wurden unter Feuchtigkeitsausschuß 5 h bei Raumtemperatur gerührt, der gebildete Feststoff mit 10 ml absolutem n-Heptan ausgerührt, abgesaugt und aus Toluol umkristallisiert.
Ausbeute 1,67 g (92 %)
Fp. 200 -201 °C

### Beispiel 2

N-4-(3-Chlor-5-(2-chlor-1,1,2-trifluorethoxy)-2-pyridyloxy)-3,5-dichlorphenyl)-N'-2,6-difluorbenzoylharnstoff

Diese Verbindung wurde analog der in Beispiel 1 beschriebenen Verfahrensweise hergestellt.
Fp. 190 -192°C

### Beispiel 3

N-((4-(3-Chlor-5-difluormethoxy-2-pyridyloxy)-3,5-dichlorphenyl)carbamoyl)-2,6-difluorbenzcarboximidsäureethylester

0,8 g (2,2 mmol) 4-(3-Chlor-5-difluormethoxy-2-pyridyloxy)-3,5-dichlorphenylisocyanat und 0,4 g (2,2 mmol) 2,6-Difluorbenzcarboximidsäureethylester wurden 2 h bei Raumtemperatur unter Feuchtigkeitsausschluß gerührt, der gebildete Fststoff mit 5 ml absolutem n-Heptan ausgerührt, abgesaugt und aus n-Heptan umkristallisiert.
Ausbeute 1,0 g (83 %)
Fp. 135 -137 °C

### Beispiel 4

5-(4-(3-Chlor-5-difluormethoxy-2-pyridyloxy)-3,5-dichlorphenyl)-2-(2,6-difluorphenyl)-6H-1,3,5-oxdiazin-4,6-dion

0,8 g (2,2 mmol) 4-(3-Chlor-5-difluormethoxy-2-pyridyloxy)-3,5-dichlorphenylisocyanat und 0,4g(2,2 mmol) 2,6-Di fluorbenzoylisocyanat wurden 16 h bei 70 °C unter Feuchtigkeitsausschluß gerührt, nach dem Abkühlen der gebildete Feststoff mit 5 ml absolutem n-Hepten ausgerührt, abgesaugt und aus Cyclohexen/Toluol umkristallisiert; Fp 154-6 °C, Ausbeute: 86 %.

Die Verbindungen der nachfolgenden Tabellen lassen sich, wie in Beispielen 1-4 beschrieben, herstellen.

**Tabelle 1**

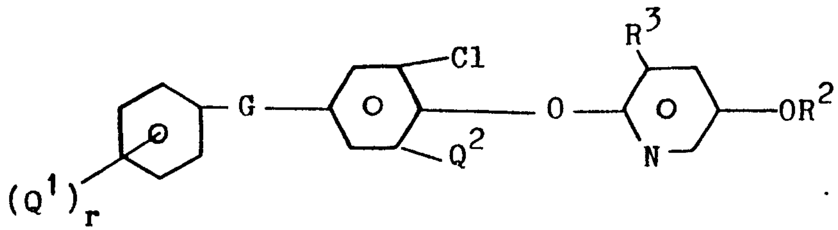

| Bsp. No. | $(Q^1)_r$ | G | $Q^2$ | $R^2$ | $R^3$ | Fp. |
|---|---|---|---|---|---|---|
| 5 | 2-F | $-\overset{O}{\overset{\|}{C}}-NH-\overset{O}{\overset{\|}{C}}-NH-$ | Cl | $CF_3$ | F | |
| 6 | $2,6F_2$ | " | $CH_3$ | $CF_3$ | F | |
| 7 | $2,4,6-Cl_3$ | " | Cl | $CF_2H$ | Cl | |
| 8 | $3,6-F_2$ | $-\overset{OC_2H_5}{\overset{\mid}{C}}=N-\overset{O}{\overset{\|}{C}}-NH-$ | Cl | $CF_3$ | F | |
| 9 | $2,4-Cl_2$ | " | $CH_3$ | H | Br | |
| 10 | 2-F | " | Cl | $CF_2CHFCF_3$ | H | |
| 11 | $2,6F_2-4-Cl$ | " | Cl | " | F | |

Fortsetzung von Tabelle 1

| Bsp. No | $(Q^1)_r$ | G | $Q^2$ | $R^2$ | $R^3$ | Fp. |
|---|---|---|---|---|---|---|
| 12 | 2-Cl | $-\overset{O}{\overset{\|}{C}}-NH-\overset{O}{\overset{\|}{C}}-NH-$ | $CH_3$ | $CH_2CF_3$ | H | |
| 13 | 2F | 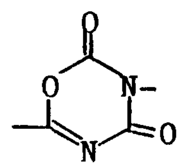 | Cl | $CF_2CF_3$ | F | |
| 14 | 2,6-$F_2$ | " | Cl | $CF_3$ | Cl | |
| 15 | 2F,6-Cl | " | Cl | $CF_2CHFBr$ | F | |

Tabelle 2

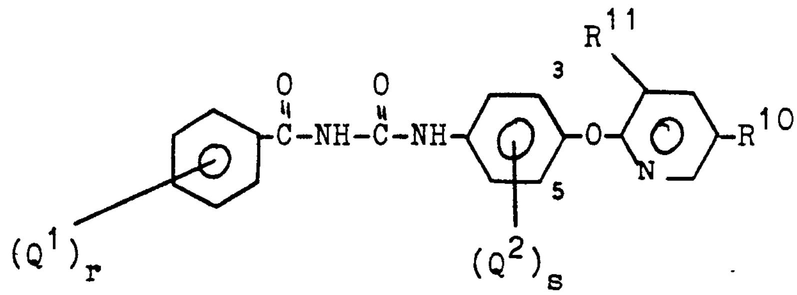

| Bsp. No. | $(Q^1)_r$ | $(Q^2)_s$ | $R^{10}$ | $R^{11}$ | Fp (°C) |
|---|---|---|---|---|---|
| 16 | 2-F | 2,5-$Cl_2$ | -$OCHF_2$ | Cl | |
| 17 | 2-Cl | 2,5-$Cl_2$ | " | Cl | 219-220 |
| 18 | 2,6-$F_2$ | 2,5-$Cl_2$ | " | Cl | 188-189 |
| 19 | 2,4-$Cl_2$ | 2,5-$Cl_2$ | " | Cl | |
| 20 | 2-$OCH_3$ | 2,5-$Cl_2$ | " | Cl | |
| 21 | 2-$CH_3$ | 2,5-$Cl_2$ | " | Cl | |
| 22 | 2-Cl | 2,5-$Cl_2$ | " | F | |
| 23 | 2,6-$F_2$ | 2,5-$Cl_2$ | " | F | |
| 24 | 2-Cl | 2,5-$Cl_2$ | " | Br | |
| 25 | 2-F | 2,5-$Cl_2$ | " | Br | |
| 26 | 2,6-$F_2$ | 2,5-$Cl_2$ | " | Br | |
| 27 | 2-Cl | 2,5-$Cl_2$ | -$OCClF_2$ | Cl | |
| 28 | 2,6-$F_2$ | 2,5-$Cl_2$ | -$OCClF_2$ | Cl | |
| 29 | 2-Cl | 2,5-$Cl_2$ | $OCF_3$ | Cl | |
| 30 | 2-F | 2,5-$Cl_2$ | " | Cl | |
| 31 | 2,6-$F_2$ | 2,5-$Cl_2$ | " | Cl | 191-193 |
| 32 | 2-Cl | 2,5-$Cl_2$ | " | F | |
| 33 | 2-F | 2,5-$Cl_2$ | " | F | |
| 34 | 2,6-$F_2$ | 2,5-$Cl_2$ | " | F | |
| 35 | 2-Cl | 2,5-$Cl_2$ | " | Br | |
| 36 | 2-F | 2,5-$Cl_2$ | " | Br | |
| 37 | 2,6-$F_2$ | 2,5-$Cl_2$ | " | Br | |
| 38 | 2-Cl | 2,5-$Cl_2$ | $OCF_2CHF_2$ | Cl | |
| 39 | 2-F | 2,5-$Cl_2$ | $OCF_2CHF_2$ | Cl | |
| 40 | 2,6-$F_2$ | 2,5-$Cl_2$ | $OCF_2CHF_2$ | Cl | 179-180 |
| 41 | 2-Cl | 2,5-$Cl_2$ | $OCF_2CHF_2$ | F | |
| 42 | 2-F | 2,5-$Cl_2$ | $OCF_2CHF_2$ | F | |

Fortsetzung von Tabelle 2

| Bsp. No. | $(Q^1)_r$ | $(Q^2)_s$ | $R^{10}$ | $R^{11}$ | Fp (°C) |
|---|---|---|---|---|---|
| 43 | 2,6-$F_2$ | 2,5-$Cl_2$ | $-OCF_2CHF_2$ | F | |
| 44 | 2-Cl | 2,5-$Cl_2$ | $-OCF_2CHF_2$ | Br | |
| 45 | 2-F | 2,5-$Cl_2$ | $-OCF_2CHF_2$ | Br | |
| 46 | 2,6-$F_2$ | 2,5-$Cl_2$ | $-OCF_2CHF_2$ | Br | |
| 47 | 2-Cl | 2,5-$Cl_2$ | $-OCF_2CHClF$ | Cl | 219-220 |
| 48 | 2,6-$F_2$ | 2,5-$Cl_2$ | $-OCF_2CHClF$ | Cl | 199-200 |
| 49 | 2-Cl | 2,5-$Cl_2$ | $-OCF_2CHClF$ | F | |
| 50 | 2,6-$F_2$ | 2,5-$Cl_2$ | $-OCF_2CHClF$ | F | |
| 51 | 2-Cl | 2,5-$Cl_2$ | $-OCF_2CHClF$ | Br | |
| 52 | 2,6-$F_2$ | 2,5-$Cl_2$ | $-OCF_2CHClF$ | Br | |
| 53 | 2,6-$F_2$ | 2,5-$Cl_2$ | $-OCF_2CHBrF$ | Cl | 185-186 |
| 54 | 2-Cl | 2,5-$Cl_2$ | $-OCF_2CHFCF_3$ | Cl | 192-193 |
| 55 | 2-F | 2,5-$Cl_2$ | $-OCF_2CHFCF_3$ | Cl | 183-184 |
| 56 | 2,6-$F_2$ | 2,5-$Cl_2$ | $-OCF_2CHFCF_3$ | Cl | 196-197 |
| 57 | 2-$CH_3$ | 2,5-$Cl_2$ | $-OCF_2CHFCF_3$ | Cl | |
| 58 | 2-$OCH_3$ | 2,5-$Cl_2$ | $-OCF_2CHFCF_3$ | Cl | |
| 59 | 2-Cl | 2,5-$Cl_2$ | $-OCF_2CHFCF_3$ | F | |
| 60 | 2-F | 2,5-$Cl_2$ | $-OCF_2CHFCF_3$ | F | |
| 61 | 2,6-$F_2$ | 2,5-$Cl_2$ | $-OCH_2CHFCF_3$ | F | |
| 62 | 2-Cl | 2,5-$Cl_2$ | $-OCH_2CHFCF_3$ | Br | |
| 63 | 2-F | 2,5-$Cl_2$ | $-OCH_2CHFCF_3$ | Br | |
| 64 | 2,6-$F_2$ | 2,5-$Cl_2$ | $-OCH_2CHFCF_3$ | Br | |
| 65 | 2-Cl | 2,5-$Cl_2$ | $-OCH_2CH=CHCl$ | Cl | 132-133 |
| 66 | 2,6-$F_2$ | 2,5-$Cl_2$ | $-OCH_2CH=CHCl$ | Cl | |
| 67 | 2-Cl | 2,5-$Cl_2$ | $-OCH_2\underset{Cl}{C} = \underset{Cl}{CH}$ | Cl | 135-136 |
| 68 | 2,6-$F_2$ | 2,5-$Cl_2$ | $-OCH_2\underset{Cl}{C} = \underset{Cl}{CH}$ | Cl | |
| 69 | 2-Cl | 2,5-$Cl_2$ | $-OCH_2C{\equiv}C-Cl$ | Cl | |
| 70 | 2,6-$F_2$ | 2,5-$Cl_2$ | $-OCH_2C{\equiv}C-Cl$ | Cl | 129-130 |
| 71 | 2-Cl | 3,5-$Cl_2$ | $-OCHF_2$ | Cl | 161-163 |
| 72 | 2-F | 3,5-$Cl_2$ | $-OCHF_2$ | Cl | 150-153 |

Fortsetzung von Tabelle 2

| Bsp. No. | $(Q^1)_r$ | $(Q^2)_s$ | $R^{10}$ | $R^{11}$ | Fp (°C) |
|---|---|---|---|---|---|
| 73 | 2-$CH_3$ | 3,5-$Cl_2$ | -$OCHF_2$ | Cl | |
| 74 | 2-$OCH_3$ | 3,5-$Cl_2$ | -$OCHF_2$ | Cl | |
| 75 | 2-Cl | 3,5-$Cl_2$ | -$OCHF_2$ | F | |
| 76 | 2-F | 3,5-$Cl_2$ | -$OCHF_2$ | F | |
| 77 | 2,6-$F_2$ | 3,5-$Cl_2$ | -$OCHF_2$ | F | |
| 78 | 2-Cl | 3,5-$Cl_2$ | -$OCHF_2$ | Br | |
| 79 | 2-F | 3,5-$Cl_2$ | -$OCHF_2$ | Br | |
| 80 | 2,6-$F_2$ | 3,5-$Cl_2$ | -$OCHF_2$ | Br | |
| 81 | 2-Cl | 3,5-$Cl_2$ | -$OCClF_2$ | Cl | 233-235 |
| 82 | 2-F | 3,5-$Cl_2$ | -$OCClF_2$ | Cl | 178-179 |
| 83 | 2,6-$F_2$ | 3,5-$Cl_2$ | -$OCClF_2$ | Cl | 203-205 |
| 84 | 2-Cl | 3,5-$Cl_2$ | -$OCClF_2$ | F | |
| 85 | 2,6-$F_2$ | 3,5-$Cl_2$ | -$OCClF_2$ | F | |
| 86 | 2-Cl | 3,5-$Cl_2$ | -$OCClF_2$ | Br | |
| 87 | 2,6-$F_2$ | 3,5-$Cl_2$ | -$OCClF_2$ | Br | |
| 88 | 2-Cl | 3,5-$Cl_2$ | -$OCF_3$ | Cl | 217-218 |
| 89 | 2-F | 3,5-$Cl_2$ | -$OCF_3$ | Cl | 201-202 |
| 90 | 2,6-$F_2$ | 3,5-$Cl_2$ | -$OCF_3$ | Cl | 198-199 |
| 91 | 2-$CH_3$ | 3,5-$Cl_2$ | -$OCF_3$ | Cl | 212-213 |
| 92 | 2-$OCH_3$ | 3,5-$Cl_2$ | -$OCF_3$ | Cl | 179-180 |
| 93 | 2-Cl | 3,5-$Cl_2$ | -$OCF_3$ | F | |
| 94 | 2-F | 3,5-$Cl_2$ | -$OCF_3$ | F | |
| 95 | 2,6-$F_2$ | 3,5-$Cl_2$ | -$OCF_3$ | F | |
| 96 | 2-$CH_3$ | 3,5-$Cl_2$ | -$OCF_3$ | F | |
| 97 | 2-$OCH_3$ | 3,5-$Cl_2$ | -$OCF_3$ | F | |
| 98 | 2-Cl | 3,5-$Cl_2$ | -$OCF_3$ | Br | 185-186 |
| 99 | 2-F | 3,5-$Cl_2$ | -$OCF_3$ | Br | 177-178 |
| 100 | 2,6-$F_2$ | 3,5-$Cl_2$ | -$OCF_3$ | Br | 201-202 |
| 101 | 2-$CH_3$ | 3,5-$Cl_2$ | -$OCF_3$ | Br | |
| 102 | 2-$OCH_3$ | 3,5-$Cl_2$ | -$OCF_3$ | Br | |
| 103 | 2-Cl | 3,5-$Cl_2$ | -$OCH_2CHF_2$ | Cl | 221-223 |

Fortsetzung von Tabelle 2

| Bsp. No. | $(Q^1)_r$ | $(Q^2)_s$ | $R^{10}$ | $R^{11}$ | Fp (°C) |
|---|---|---|---|---|---|
| 104 | 2-F | 3,5-$Cl_2$ | $-OCH_2CHF_2$ | Cl | 164-165 |
| 105 | 2,6-$F_2$ | 3,5-$Cl_2$ | $-OCH_2CHF_2$ | Cl | 193-195 |
| 106 | 2-Cl | 3,5-$Cl_2$ | $-OCH_2CHF_2$ | F | |
| 107 | 2-F | 3,5-$Cl_2$ | $-OCH_2CHF_2$ | F | |
| 108 | 2,6-$F_2$ | 3,5-$Cl_2$ | $-OCH_2CHF_2$ | F | |
| 109 | 2-Cl | 3,5-$Cl_2$ | $-OCH_2CHF_2$ | Br | |
| 110 | 2-F | 3,5-$Cl_2$ | $-OCH_2CHF_2$ | Br | |
| 111 | 2,6-$F_2$ | 3,5-$Cl_2$ | $-OCH_2CHF_2$ | Br | |
| 112 | 2-Cl | 3,5-$Cl_2$ | $-OCF_2CF_3$ | Cl | |
| 113 | 2,6-$F_2$ | 3,5-$Cl_2$ | $-OCF_2CF_3$ | Cl | |
| 114 | 2-Cl | 3,5-$Cl_2$ | $-OCF_2CCl_2F$ | Cl | |
| 115 | 2-F | 3,5-$Cl_2$ | $-OCF_2CCl_2F$ | Cl | |
| 116 | 2-Cl | 3,5-$Cl_2$ | $-OCF_2CHClF$ | Cl | 228-229 |
| 117 | 2-F | 3,5-$Cl_2$ | $-OCF_2CHClF$ | Cl | 170-171 |
| 118 | 2,4-$Cl_2$ | 3,5-$Cl_2$ | $-OCF_2CHClF$ | Cl | |
| 119 | 2-$CH_3$ | 3,5-$Cl_2$ | $-OCF_2CHClF$ | Cl | |
| 120 | 2-$OCH_3$ | 3,5-$Cl_2$ | $-OCF_2CHClF$ | Cl | |
| 121 | 2-Cl | 3,5-$Cl_2$ | $-OCF_2CHClF$ | F | |
| 122 | 2,6-$F_2$ | 3,5-$Cl_2$ | $-OCF_2CHClF$ | F | |
| 123 | 2-Cl | 3,5-$Cl_2$ | $-OCF_2CHClF$ | Br | |
| 124 | 2,6-$F_2$ | 3,5-$Cl_2$ | $-OCF_2CHClF$ | Br | |
| 125 | 2,6-$F_2$ | 3,5-$Cl_2$ | $-OCF_2CHBrF$ | Cl | 191-192 |
| 126 | 2-Cl | 3,5-$Cl_2$ | $-OCF_2CHFCF_3$ | Cl | 216-217 |
| 127 | 2-F | 3,5-$Cl_2$ | $-OCF_2CHFCF_3$ | Cl | 178-179 |
| 128 | 2,6-$F_2$ | 3,5-$Cl_2$ | $-OCF_2CHFCF_3$ | Cl | 189-190 |
| 129 | 2-$CH_3$ | 3,5-$Cl_2$ | $-OCF_2CHFCF_3$ | Cl | |
| 130 | 2-$OCH_3$ | 3,5-$Cl_2$ | $-OCF_2CHFCF_3$ | Cl | |
| 131 | 2-Cl | 3,5-$Cl_2$ | $-OCF_2CHFCF_3$ | F | |
| 132 | 2-F | 3,5-$Cl_2$ | $-OCF_2CHFCF_3$ | F | |
| 133 | 2,6-$F_2$ | 3,5-$Cl_2$ | $-OCF_2CHFCF_3$ | F | |
| 134 | 2-Cl | 3,5-$Cl_2$ | $-OCF_2CHFCF_3$ | Br | |
| 135 | 2-F | 3,5-$Cl_2$ | $-OCF_2CHFCF_3$ | Br | |

Fortsetzung von Tabelle 2

| Bsp. No. | $(Q^1)_r$ | $(Q^2)_s$ | $R^{10}$ | $R^{11}$ | Fp (°C) |
|---|---|---|---|---|---|
| 136 | $2,6-F_2$ | $3,5-Cl_2$ | $-OCF_2CHFCF_3$ | Br | |
| 137 | 2-Cl | $3,5-Cl_2$ | $-OCH_2CH=CHCl$ | Cl | |
| 138 | 2-F | $3,5-Cl_2$ | $-OCH_2CH=CHCl$ | Cl | |
| 139 | $2,6-F_2$ | $3,5-Cl_2$ | $-OCH_2CH=CHCl$ | Cl | 136-137 |
| 140 | 2-Cl | $3,5-Cl_2$ | $-OCH_2\underset{Cl}{C}=\underset{Cl}{CH}$ | Cl | |
| 141 | 2-F | $3,5-Cl_2$ | $-OCH_2\underset{Cl}{C}=\underset{Cl}{CH}$ | Cl | |
| 142 | $2,6-F_2$ | $3,5-Cl_2$ | $-OCH_2\underset{Cl}{C}=\underset{Cl}{CH}$ | Cl | 129-130 |
| 143 | 2-Cl | $3,5-Cl_2$ | $-OCH_2C\equiv C-Cl$ | Cl | |
| 144 | $2,6-F_2$ | $3,5-Cl_2$ | $-OCH_2C\equiv C-Cl$ | Cl | 139-140 |
| 145 | 2-Cl | $3,5-Cl_2$ | Cl | $OCHF_2$ | 183-184 |
| 146 | 2-F | $3,5-Cl_2$ | Cl | $-OCHF_2$ | |
| 147 | $2,6-F_2$ | $3,5-Cl_2$ | Cl | $-OCHF_2$ | 219-220 |
| 148 | 2-Cl | $3,5-Cl_2$ | Cl | $-OCF_2CHClF$ | 215-216 |
| 149 | $2,6-F_2$ | $3,5-Cl_2$ | Cl | $-OCF_2CHClF$ | 193-194 |
| 150 | 2-Cl | $3,5-Cl_2$ | Cl | $-OCF_2CHF_2$ | |
| 151 | $2,6-F_2$ | $3,5-Cl_2$ | Cl | $-OCF_2CHF_2$ | |
| 152 | 2-Cl | $3,5-Cl_2$ | Cl | $-OCF_2CHFCF_3$ | |
| 153 | $2,6-F_2$ | $3,5-Cl_2$ | Cl | $-OCF_2CHFCF_3$ | |
| 154 | 2-Cl | $3,5-Cl_2$ | $-OCHF_2$ | H | |
| 155 | $2,6-F_2$ | $3,5-Cl_2$ | $-OCHF_2$ | H | |
| 156 | 2-Cl | $3,5-Cl_2$ | $-OCF_3$ | H | |
| 157 | $2,6-F_2$ | $3,5-Cl_2$ | $-OCF_3$ | H | 173-174 |
| 158 | 2-Cl | $3,5-Cl_2$ | $-OCF_2CHF_2$ | H | |
| 159 | $2,6-F_2$ | $3,5-Cl_2$ | $-OCF_2CHF_2$ | H | |
| 160 | 2-Cl | $3,5-Cl_2$ | $-OCF_2CHFCF_3$ | H | 193-194 |
| 161 | $2,6-F_2$ | $3,5-Cl_2$ | $-OCF_2CHFCF_3$ | Cl | |
| 162 | 2-Cl | $3,5-Cl_2$ | $-OCHF_2$ | Cl | |
| 163 | 2-F | $3,5-Cl_2$;2-F | $-OCHF_2$ | Cl | |
| 164 | $2,6-F_2$ | $3,5-Cl_2$;2-F | $-OCHF_2$ | Cl | |
| 165 | 2-Cl | $3,5-Cl_2$;2-F | $-OCF_3$ | Cl | |

Fortsetzung von Tabelle 2

| Bsp. No. | $(Q^1)_r$ | $(Q^2)_s$ | $R^{10}$ | $R^{11}$ | Fp (°C) |
|---|---|---|---|---|---|
| 166 | 2-F | 3,5-$Cl_2$;2-F | $-OCF_3$ | Cl | |
| 167 | 2,6-$F_2$ | 3,5-$Cl_2$;2-F | $-OCF_3$ | Cl | |
| 168 | 2-Cl | 3,5-$Cl_2$;2-F | $-OCF_2CHF_2$ | Cl | |
| 169 | 2,6-$F_2$ | 3,5-$Cl_2$;2-F | $-OCF_2CHF_2$ | Cl | |
| 170 | 2-Cl | 3,5-$Cl_2$;2-F | $-OCF_2CHFCF_3$ | Cl | |
| 171 | 2-F | 3,5-$Cl_2$;2-F | $-OCF_2CHFCF_3$ | Cl | |
| 172 | 2,6-$F_2$ | 3,5-$Cl_2$;2-F | $-OCF_2CHFCF_3$ | Cl | |
| 173 | 2-Cl | 2-Cl;5-$CF_3$ | $-OCHF_2$ | Cl | |
| 174 | 2-F | 2-Cl;5-$CF_3$ | $-OCHF_2$ | Cl | |
| 175 | 2,6-$F_2$ | 2-Cl;5-$CF_3$ | $-OCHF_2$ | Cl | 195-196 |
| 176 | 2-Cl | 2-Cl;5-$CF_3$ | $-OCF_3$ | Cl | |
| 177 | 2-F | 2-Cl;5-$CF_3$ | $-OCF_3$ | Cl | |
| 178 | 2,6-$F_2$ | 2-Cl;5-$CF_3$ | $-OCF_3$ | Cl | 201-203 |
| 179 | 2-Cl | 2-Cl;5-$CF_3$ | $-OCF_2CHF_2$ | Cl | |
| 180 | 2-F | 2-Cl;5-$CF_3$ | $-OCF_2CHF_2$ | Cl | |
| 181 | 2,6-$F_2$ | 2-Cl;5-$CF_3$ | $-OCF_2CHF_2$ | Cl | |
| 182 | 2,6-$F_2$ | 2-Cl;5-$CF_3$ | $-OCF_2CHClF$ | Cl | |
| 183 | 2-Cl | 2-Cl;5-$CF_3$ | $-OCF_2CHFCF_3$ | Cl | |
| 184 | 2-F | 2-Cl;5-$CF_3$ | $-OCF_2CHFCF_3$ | Cl | |
| 185 | 2,6-$F_2$ | 2-Cl;5-$CF_3$ | $-OCF_2CHFCF_3$ | Cl | 192-193 |
| 186 | 2-Cl | 3-$CF_3$ | $-OCHF_2$ | Cl | |
| 187 | 2-F | 3-$CF_3$ | $-OCHF_2$ | Cl | |
| 188 | 2,6-$F_2$ | 3-$CF_3$ | $-OCHF_2$ | Cl | |
| 189 | 2-Cl | 3-$CF_3$ | $-OCF_3$ | Cl | |
| 190 | 2-F | 3-$CF_3$ | $-OCF_3$ | Cl | |
| 191 | 2,6-$F_2$ | 3-$CF_3$ | $-OCF_3$ | Cl | |
| 192 | 2-Cl | 3-$CF_3$ | $-OCF_2CHF_2$ | Cl | |
| 193 | 2-F | 3-$CF_3$ | $-OCF_2CHF_2$ | Cl | |
| 194 | 2,6-$F_2$ | 3-$CF_3$ | $-OCF_2CHF_2$ | Cl | |
| 195 | 2-Cl | 3-$CF_3$ | $-OCF_2CHClF$ | Cl | |
| 196 | 2-F | 3-$CF_3$ | $-OCF_2CHClF$ | Cl | |
| 197 | 2,6-$F_2$ | 3-$CF_3$ | $-OCF_2CHClF$ | Cl | |
| 198 | 2-Cl | 3-$CF_3$ | $-OCF_2CHFCF_3$ | Cl | 192-194 |

Fortsetzung von Tabelle 2

| Bsp. No. | $(Q^1)_r$ | $(Q^2)_s$ | $R^{10}$ | $R^{11}$ | Fp (°C) |
|---|---|---|---|---|---|
| 199 | 2-F | $3-CF_3$ | $-OCF_2CHFCF_3$ | Cl | |
| 200 | $2,6-F_2$ | $3-CF_3$ | $-OCF_2CHFCF_3$ | Cl | |
| 201 | 2-Cl | $3-COOCH_3$ | $-OCHF_2$ | Cl | |
| 202 | 2-F | $3-COOCH_3$ | $-OCHF_2$ | Cl | |
| 203 | $2,6-F_2$ | $3-COOCH_3$ | $-OCHF_2$ | Cl | |
| 204 | 2-Cl | $3-COOCH_3$ | $-OCF_3$ | Cl | |
| 205 | 2-F | $3-COOCH_3$ | $-OCF_3$ | Cl | |
| 206 | $2,6-F_2$ | $3-COOCH_3$ | $-OCF_3$ | Cl | 211-212 |
| 207 | 2-Cl | $3-COOCH_3$ | $-OCF_2CHF_2$ | Cl | |
| 208 | 2-F | $3-COOCH_3$ | $-OCF_2CHF_2$ | Cl | |
| 209 | $2,6-F_2$ | $3-COOCH_3$ | $-OCF_2CHF_2$ | Cl | |
| 210 | 2-Cl | $3-COOCH_3$ | $-OCF_2CHClF$ | Cl | |
| 211 | 2-F | $3-COOCH_3$ | $-OCF_2CHClF$ | Cl | |
| 212 | $2,6-F_2$ | $3-COOCH_3$ | $-OCF_2CHClF$ | Cl | |
| 213 | 2-Cl | $3-COOCH_3$ | $-OCF_2CHFCF_3$ | Cl | |
| 214 | 2-F | $3-COOCH_3$ | $-OCF_2CHFCF_3$ | Cl | |
| 215 | $2,6-F_2$ | $3-COOCH_3$ | $-OCF_2CHFCF_3$ | Cl | 203-204 |
| 216 | 2-Cl | $3-Cl;5-COOCH_3$ | $-OCHF_2$ | Cl | |
| 217 | 2-F | $3-Cl;5-COOCH_3$ | $-OCHF_2$ | Cl | |
| 218 | $2,6-F_2$ | $3-Cl;5-COOCH_3$ | $-OCHF_2$ | Cl | |
| 219 | 2-Cl | $3-Cl;5-COOCH_3$ | $-OCF_3$ | Cl | |
| 220 | 2-F | $3-Cl;5-COOCH_3$ | $-OCF_3$ | Cl | |
| 221 | $2,6-F_2$ | $3-Cl;5-COOCH_3$ | $-OCF_3$ | Cl | |
| 222 | 2-Cl | $3-Cl;5-COOCH_3$ | $-OCF_2CHF_2$ | Cl | |
| 223 | 2-F | $3-Cl;5-COOCH_3$ | $-OCF_2CHF_2$ | Cl | |
| 224 | $2,6-F_2$ | $3-Cl;5-COOCH_3$ | $-OCF_2CHF_2$ | Cl | |
| 225 | 2-Cl | $3-Cl;5-COOCH_3$ | $-OCF_2CHClF$ | Cl | |
| 226 | 2-F | $3-Cl;5-COOCH_3$ | $-OCF_2CHClF$ | Cl | |
| 227 | $2,6-F_2$ | $3-Cl;5-COOCH_3$ | $-OCF_2CHClF$ | Cl | |
| 228 | 2-Cl | $3-Cl;5-COOCH_3$ | $-OCF_2CHFCF_3$ | Cl | |
| 229 | 2-F | $3-Cl;5-COOCH_3$ | $-OCF_2CHFCF_3$ | Cl | |
| 230 | $2,6-F_2$ | $3-Cl;5-COOCH_3$ | $-OCF_2CHFCF_3$ | Cl | |

Tabelle 3

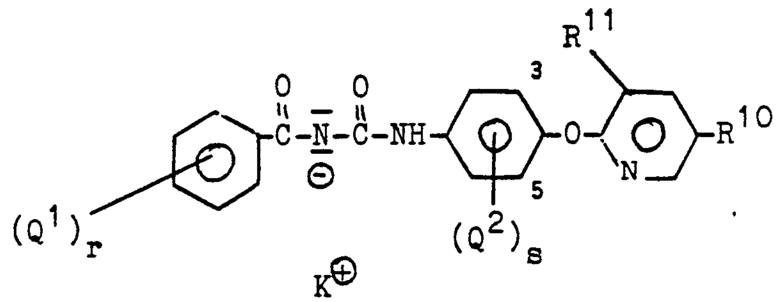

| Bsp. No. | $(Q^1)_r$ | K | $(Q^2)_s$ | $R^{10}$ | $R^{11}$ | Fp (°C) |
|---|---|---|---|---|---|---|
| 231 | 2,6-$F_2$ | Na | 3,5-$Cl_2$ | $-OCHF_2$ | Cl | |
| 232 | " | Na | " | $-OCF_3$ | " | |
| 233 | " | Na | " | $-OCF_2CHF_2$ | " | |
| 234 | " | Na | " | $-OCF_2CHClF$ | " | |
| 235 | " | Na | " | $-OCF_2CHFCF_3$ | " | 104-105 (Z.) |
| 236 | " | $N(C_4H_9)_4$ | " | $-OCHF_2$ | " | |
| 237 | " | " | " | $-OCF_3$ | " | |
| 238 | " | " | " | $-OCF_2CHF_2$ | " | |
| 239 | " | " | " | $-OCF_2CHClF$ | " | |
| 240 | " | " | " | $-OCF_2CHFCF_3$ | " | |

Tabelle 4

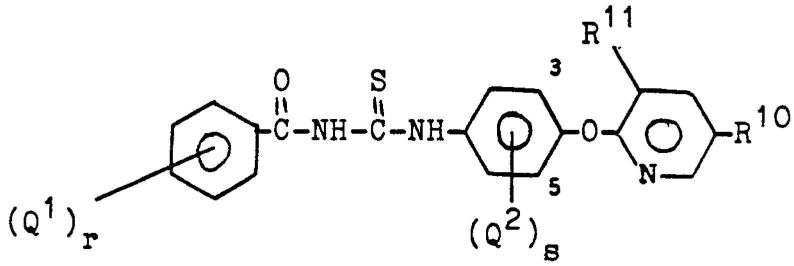

| Bsp. No. | $(Q^1)_r$ | $(Q^2)_s$ | $R^{10}$ | $R^{11}$ | Fp (°C) |
|---|---|---|---|---|---|
| 241 | 2-Cl | 3,5-$Cl_2$ | -$OCHF_2$ | Cl | |
| 242 | 2-F | " | " | " | |
| 243 | 2,6-$F_2$ | " | " | " | 228-230 |
| 244 | 2-Cl | " | -$OCF_3$ | " | |
| 245 | 2-F | " | " | " | |
| 246 | 2,6-$F_2$ | " | " | " | 219-220 |
| 247 | 2-Cl | " | -$OCF_2CHF_2$ | " | |
| 248 | 2-F | " | " | " | |
| 249 | 2,6-$F_2$ | " | " | " | 214-215 |
| 250 | 2-Cl | " | -$OCF_2CHClF$ | " | |
| 251 | 2-F | " | " | " | |
| 252 | 2,6-$F_2$ | " | " | " | |
| 253 | 2-Cl | " | -$OCF_2CHFCF_3$ | " | |
| 254 | 2-F | " | " | " | |
| 255 | 2,6-$F_2$ | " | " | " | 223-225 |

Tabelle 5

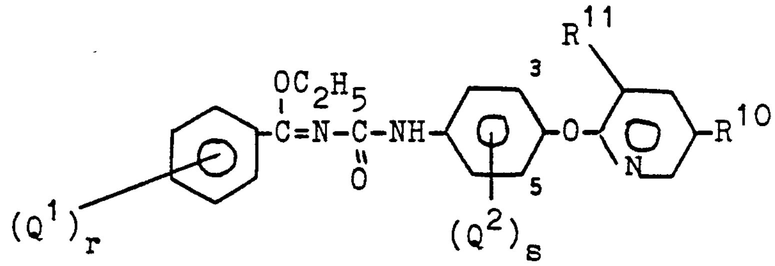

| Bsp. No. | $(Q^1)_r$ | $(Q^2)_s$ | $R^{10}$ | $R^{11}$ | Fp (°C) |
|---|---|---|---|---|---|
| 256 | 2-Cl | 3,5-$Cl_2$ | $-OCHF_2$ | Cl | |
| 257 | 2-F | 3,5-$Cl_2$ | $-OCHF_2$ | Cl | |
| 258 | 2,4-$Cl_2$ | 3,5-$Cl_2$ | $-OCHF_2$ | Cl | |
| 259 | 2-Cl | 3,5-$Cl_2$ | $-OCF_3$ | Cl | 109-110 |
| 260 | 2-F | 3,5-$Cl_2$ | $-OCF_3$ | Cl | |
| 261 | 2,6-$F_2$ | 3,5-$Cl_2$ | $-OCF_3$ | Cl | 128-129 |
| 262 | 2-Cl | 3,5-$Cl_2$ | $-OCF_2CHF_2$ | Cl | |
| 263 | 2-F | 3,5-$Cl_2$ | $-OCF_2CHF_2$ | Cl | |
| 264 | 2,6-$F_2$ | 3,5-$Cl_2$ | $-OCF_2CHF_2$ | Cl | |
| 265 | 2-Cl | 3,5-$Cl_2$ | $-OCF_2CHClF$ | Cl | |
| 266 | 2-F | 3,5-$Cl_2$ | $-OCF_2CHClF$ | Cl | |
| 267 | 2,6-$F_2$ | 3,5-$Cl_2$ | $-OCF_2CHClF$ | Cl | |
| 268 | 2-Cl | 3,5-$Cl_2$ | $-OCF_2CHFCF_3$ | Cl | 123-124 |
| 269 | 2-F | 3,5-$Cl_2$ | $-OCF_2CHFCF_3$ | Cl | |
| 270 | 2,6-$F_2$ | 3,5-$Cl_2$ | $-OCF_2CHFCF_3$ | Cl | 119-120 |

Tabelle 6

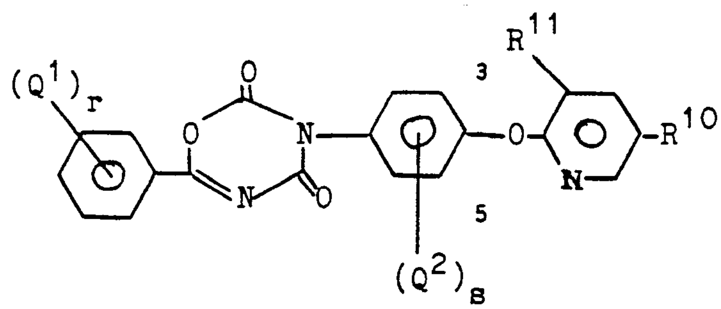

| Bsp. No. | $(Q^1)_r$ | $(Q^2)_s$ | $R^{10}$ | $R^{11}$ | Fp (°C) |
|---|---|---|---|---|---|
| 271 | 2-Cl | 3,5-$Cl_2$ | -$OCHF_2$ | Cl | |
| 272 | 2-F | 3,5-$Cl_2$ | -$OCHF_2$ | Cl | |
| 273 | 2,4-$Cl_2$ | 3,5-$Cl_2$ | -$OCHF_2$ | Cl | |
| 274 | 2-Cl | 3,5-$Cl_2$ | -$OCF_3$ | Cl | |
| 275 | 2-F | 3,5-$Cl_2$ | -$OCF_3$ | Cl | |
| 276 | 2,6-$F_2$ | 3,5-$Cl_2$ | -$OCF_3$ | Cl | 193-194 |
| 277 | 2-Cl | 3,5-$Cl_2$ | -$OCF_2CHF_2$ | Cl | |
| 278 | 2-F | 3,5-$Cl_2$ | -$OCF_2CHF_2$ | Cl | |
| 279 | 2,6-$F_2$ | 3,5-$Cl_2$ | -$OCF_2CHF_2$ | Cl | 179-180 |
| 280 | 2-Cl | 3,5-$Cl_2$ | -$OCF_2CHClF$ | Cl | |
| 281 | 2-F | 3,5-$Cl_2$ | -$OCF_2CHClF$ | Cl | |
| 282 | 2,6-$F_2$ | 3,5-$Cl_2$ | -$OCF_2CHClF$ | Cl | |
| 283 | 2-Cl | 3,5-$Cl_2$ | -$OCF_2CHFCF_3$ | Cl | |
| 284 | 2-F | 3,5-$Cl_2$ | -$OCF_2CHFCF_3$ | Cl | |
| 285 | 2,6-$F_2$ | 3,5-$Cl_2$ | -$OCF_2CHFCF_3$ | Cl | 199-200 |

Tabelle 7

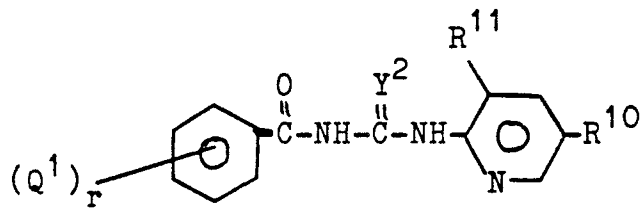

| Bsp. No. | $(Q^1)_r$ | $Y^2$ | $R^{10}$ | $R^{11}$ | Fp (°C) |
|---|---|---|---|---|---|
| 286 | 2-Cl | O | $-OCHF_2$ | Cl | |
| 287 | 2-F | O | $-OCHF_2$ | Cl | |
| 288 | 2,6-$F_2$ | O | $-OCHF_2$ | Cl | |
| 289 | 2-Cl | O | $-OCF_3$ | Cl | 129 |
| 290 | 2-F | O | $-OCF_3$ | Cl | 133-135 |
| 291 | 2,6-$F_2$ | O | $-OCF_3$ | Cl | 157 |
| 292 | 2-Cl | O | $-OCF_2CHF_2$ | Cl | |
| 293 | 2-F | O | $-OCF_2CHF_2$ | Cl | |
| 294 | 2,6-$F_2$ | O | $-OCF_2CHF_2$ | Cl | 154-155 |
| 295 | 2-Cl | O | $-OCF_2CHClF$ | Cl | |
| 296 | 2-F | O | $-OCF_2CHClF$ | Cl | |
| 297 | 2,6-$F_2$ | O | $-OCF_2CHClF$ | Cl | |
| 298 | 2-Cl | O | $-OCF_2CHFCF_3$ | Cl | |
| 299 | 2-F | O | $-OCF_2CHFCF_3$ | Cl | |
| 300 | 2,6-$F_2$ | O | $-OCF_2CHFCF_3$ | Cl | |
| 301 | 2-Cl | O | $-OCF_2CHFCF_3$ | Br | |
| 302 | 2-F | O | $-OCF_2CHFCF_3$ | Br | |
| 303 | 2,6-$F_2$ | O | $-OCF_2CHFCF_3$ | Br | 131-133 |
| 304 | 2-Cl | O | H | $-OCF_2CHFCF_3$ | 164-165 |
| 305 | 2-F | O | H | $-OCF_2CHFCF_3$ | 130-131 |
| 306 | 2,6-$F_2$ | O | H | $-OCF_2CHFCF_3$ | 138 |
| 307 | 2-Cl | S | $-OCHF_2$ | Cl | |
| 308 | 2-F | S | $-OCHF_2$ | Cl | |
| 309 | 2,6-$F_2$ | S | $-OCHF_2$ | Cl | |
| 310 | 2-Cl | S | $-OCF_3$ | Cl | |

Fortsetzung der Tabelle 7

| Bsp. No. | $(Q^1)_r$ | $Y^2$ | $R^{10}$ | $R^{11}$ | Fp (°C) |
|---|---|---|---|---|---|
| 311 | 2-F | S | $-OCF_3$ | Cl | |
| 312 | 2,6-$F_2$ | S | $-OCF_3$ | Cl | |
| 313 | 2-Cl | S | $-OCF_2CHF_2$ | Cl | |
| 314 | 2-F | S | $-OCF_2CHF_2$ | Cl | |
| 315 | 2,6-$F_2$ | S | $-OCF_2CHF_2$ | Cl | |
| 316 | 2-Cl | S | $-OCF_2CHClF$ | Cl | |
| 317 | 2-F | S | $-OCF_2CHClF$ | Cl | |
| 318 | 2,6-$F_2$ | S | $-OCF_2CHClF$ | Cl | |
| 319 | 2-Cl | S | $-OCF_2CHFCF_3$ | Cl | |
| 320 | 2-F | S | $-OCF_2CHFCF_3$ | Cl | |
| 321 | 2,6-$F_2$ | S | $-OCF_2CHFCF_3$ | Cl | |

Tabelle 8

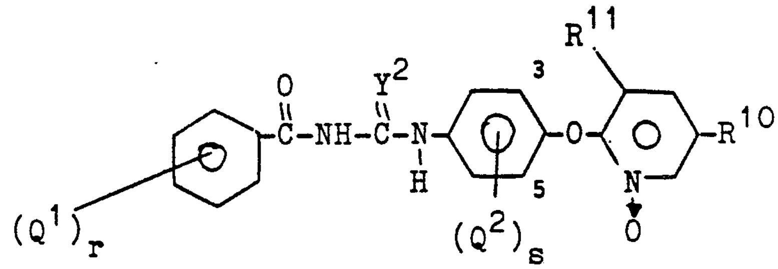

| Bsp. No. | $(Q^1)_r$ | $Y^2$ | $(Q_2)_s$ | $R^{10}$ | $R^{11}$ | Fp (°C) |
|---|---|---|---|---|---|---|
| 322 | 2-Cl | O | 2,5-$Cl_2$ | $OCHF_2$ | Cl | |
| 323 | 2-F | O | " | " | " | |
| 324 | 2,6-$F_2$ | O | " | " | " | |
| 325 | 2-Cl | O | " | $OCF_3$ | " | |
| 326 | 2-F | O | " | " | " | |
| 327 | 2,6-$F_2$ | O | " | " | " | |
| 328 | 2-Cl | O | " | $OCF_2CHF_2$ | " | |
| 339 | 2-F | O | " | " | " | |
| 330 | 2,6-$F_2$ | O | " | " | " | |
| 331 | 2-Cl | O | " | $OCF_2CHClF$ | " | |
| 332 | 2-F | O | " | " | " | |
| 333 | 2,6-$F_2$ | O | " | " | " | |
| 334 | 2-Cl | O | " | $OCF_2CHFCF_3$ | " | |
| 335 | 2-F | O | " | " | " | |
| 336 | 2,6-$F_2$ | O | " | " | " | |
| 337 | 2-Cl | O | 3,5-$Cl_2$ | $OCHF_2$ | " | |
| 338 | 2-F | O | " | " | " | |
| 339 | 2,6-$F_2$ | O | " | " | " | |
| 340 | 2-Cl | O | " | $OCF_3$ | " | |
| 341 | 2-F | O | " | " | " | |
| 342 | 2,6-$F_2$ | O | " | " | " | |
| 343 | 2-Cl | O | " | $OCF_2CHF_2$ | " | |
| 344 | 2-F | O | 3,5-$Cl_2$ | " | " | |
| 345 | 2,6-$F_2$ | O | " | " | " | |
| 346 | 2-Cl | O | " | $OCF_2CHClF$ | " | |
| 347 | 2-F | O | " | " | " | |

Fortsetzung Tabelle 8

| Bsp. No. | $(Q^1)_r$ | $Y^2$ | $(Q_2)_s$ | $R^{10}$ | $R^{11}$ | Fp (°C) |
|---|---|---|---|---|---|---|
| 348 | 2,6-$F_2$ | O | 3,5-$Cl_2$ | $OCF_2CHClF$ | Cl | |
| 349 | 2-Cl | O | " | $OCF_2CHFCF_3$ | " | |
| 350 | 2-F | O | " | " | " | |
| 351 | 2,6-$F_2$ | O | " | " | " | |
| 352 | 2-Cl | O | 3,5-$Cl_2$; 2-F | $-OCHF_2$ | " | |
| 353 | 2-F | O | " | " | " | |
| 354 | 2,6-$F_2$ | O | " | " | " | |
| 355 | 2-Cl | O | " | $-OCF_3$ | " | |
| 356 | 2-F | O | " | " | " | |
| 357 | 2,6-$F_2$ | O | " | " | " | |
| 358 | 2-Cl | O | " | $-OCF_2CHF_2$ | " | |
| 359 | 2-F | O | " | " | " | |
| 360 | 2,6-$F_2$ | O | " | " | " | |
| 361 | 2-Cl | O | " | $-OCF_2CHClF$ | " | |
| 362 | 2-F | O | " | " | " | |
| 363 | 2,6-$F_2$ | O | " | " | " | |
| 364 | 2-Cl | O | " | $OCF_2CHFCF_3$ | " | |
| 365 | 2-F | O | " | " | " | |
| 366 | 2,6-$F_2$ | O | " | " | " | |
| 367 | 2-Cl | O | 2-Cl, 5-$CF_3$ | $-OCHF_2$ | " | |
| 368 | 2-F | O | " | " | " | |
| 369 | 2,6-$F_2$ | O | " | " | " | |
| 370 | 2-Cl | O | " | $-OCF_3$ | " | |
| 371 | 2-F | O | " | " | " | |
| 372 | 2,6-$F_2$ | O | " | " | " | |
| 373 | 2-Cl | O | " | $-OCF_2CHF_2$ | " | |
| 374 | 2-F | O | " | " | " | |
| 375 | 2,6-$F_2$ | O | " | " | " | |
| 376 | 2-Cl | O | " | $-OCF_2CHClF$ | " | |
| 377 | 2-F | O | " | " | " | |
| 378 | 2,6-$F_2$ | O | " | " | " | |

Fortsetzung Tabelle 8

| Bsp. No. | $(Q^1)_r$ | $Y^2$ | $(Q_2)_s$ | $R^{10}$ | $R^{11}$ | Fp (°C) |
|---|---|---|---|---|---|---|
| 379 | 2-Cl | O | 2-Cl, 5-$CF_3$ | $-OCF_2CHFCF_3$ | Cl | |
| 380 | 2-F | O | " | " | " | |
| 381 | 2,6-$F_2$ | O | " | " | " | |
| 382 | 2-Cl | O | 3-$CF_3$ | $-OCHF_2$ | " | |
| 383 | 2-F | O | " | " | " | |
| 384 | 2,6-$F_2$ | O | " | " | " | |
| 385 | 2-Cl | O | " | $-OCF_3$ | " | |
| 386 | 2-F | O | " | " | " | |
| 387 | 2,6-$F_2$ | O | " | " | " | |
| 388 | 2-Cl | O | " | $-OCF_2CHF_2$ | " | |
| 389 | 2-F | O | " | " | " | |
| 390 | 2,6-$F_2$ | O | " | " | " | |
| 391 | 2-Cl | O | " | $-OCF_2CHClF$ | " | |
| 392 | 2-F | O | " | " | " | |
| 393 | 2,6-$F_2$ | O | " | " | " | |
| 394 | 2-Cl | O | " | $-OCF_2CHFCF_3$ | " | |
| 395 | 2-F | O | " | " | " | |
| 396 | 2,6-$F_2$ | O | " | " | " | |
| 397 | 2-Cl | O | 3-$COOCH_3$ | $-OCHF_2$ | " | |
| 398 | 2-F | O | " | " | " | |
| 399 | 2,6-$F_2$ | O | " | " | " | |
| 400 | 2-Cl | O | " | $-OCF_3$ | " | |
| 401 | 2-F | O | " | " | " | |
| 402 | 2,6-$F_2$ | O | " | " | " | |
| 403 | 2-Cl | O | " | $-OCF_2CHF_2$ | " | |
| 404 | 2-F | O | " | " | " | |
| 405 | 2,6-$F_2$ | O | " | " | " | |
| 406 | 2-Cl | O | " | $-OCF_2CHClF$ | " | |
| 407 | 2-F | O | " | " | " | |
| 408 | 2,6-$F_2$ | O | " | " | " | |

Fortsetzung Tabelle 8

| Bsp. No. | $(Q^1)_r$ | $Y^2$ | $(Q_2)_s$ | $R^{10}$ | $R^{11}$ | Fp (°C) |
|---|---|---|---|---|---|---|
| 409 | 2-Cl | O | 3-$COOCH_3$ | -$OCF_2CHFCF_3$ | Cl | |
| 410 | 2-F | O | " | " | " | |
| 411 | 2,6-$F_2$ | O | " | " | " | |
| 412 | 2-Cl | O | 3-Cl; 5-$COOCH_3$ | -$OCHF_2$ | " | |
| 413 | 2-F | O | " | " | " | |
| 414 | 2,6-$F_2$ | O | " | " | " | |
| 415 | 2-Cl | O | " | -$OCF_3$ | " | |
| 416 | 2-F | O | " | " | " | |
| 417 | 2,6-$F_2$ | O | " | " | " | |
| 418 | 2-Cl | O | " | -$OCF_2CHF_2$ | " | |
| 419 | 2-F | O | " | " | " | |
| 420 | 2,6-$F_2$ | O | " | " | " | |
| 421 | 2-Cl | O | " | -$OCF_2CHClF$ | " | |
| 422 | 2-F | O | " | " | " | |
| 423 | 2,6-$F_2$ | O | " | " | " | |
| 424 | 2-Cl | O | " | -$OCF_2CHFCF_3$ | " | |
| 425 | 2-F | O | " | " | " | |
| 426 | 2,6-$F_2$ | O | " | " | " | |
| 427 | 2-Cl | S | 3,5-$Cl_2$ | -$OCHF_2$ | " | |
| 428 | 2-F | S | " | " | " | |
| 429 | 2,6-$F_2$ | S | " | " | " | |
| 430 | 2-Cl | S | " | -$OCF_3$ | " | |
| 431 | 2-F | S | " | " | " | |
| 432 | 2,6-$F_2$ | S | " | " | " | |
| 433 | 2-Cl | S | " | -$OCF_2CHF_2$ | " | |
| 434 | 2-F | S | " | " | " | |
| 435 | 2,6-$F_2$ | S | " | " | " | |
| 436 | 2-Cl | S | " | -$OCF_2CHClF$ | " | |
| 437 | 2-F | S | " | " | " | |
| 438 | 2,6-$F_2$ | S | " | " | " | |
| 439 | 2-Cl | S | " | -$OCF_2CHFCF_2$ | " | |
| 440 | 2-F | S | " | " | " | |
| 441 | 2,6-$F_2$ | S | " | " | " | |

Tabelle 9

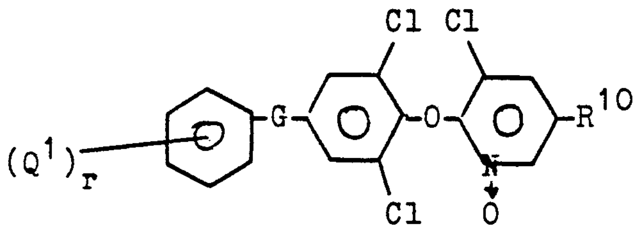

| Bsp. No. | $(Q^1)_r$ | G | $R^{10}$ | Fp (°C) |
|---|---|---|---|---|
| 442 | 2-Cl | 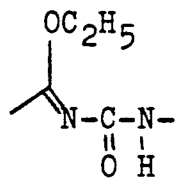 | $-OCHF_2$ | |
| 443 | 2-F | | $-OCHF_2$ | |
| 444 | 2,6-$F_2$ | | $-OCHF_2$ | |
| 445 | 2-Cl | | $-OCF_3$ | |
| 446 | 2-F | " | $-OCF_3$ | |
| 447 | 2,6-$F_2$ | " | $-OCF_3$ | |
| 448 | 2-Cl | " | $-OCF_2CHF_2$ | |
| 449 | 2-F | " | $-OCF_2CHF_2$ | |
| 450 | 2,6-$F_2$ | " | $-OCF_2CHF_2$ | |
| 451 | 2-Cl | " | $OCF_2CHClF$ | |
| 452 | 2-F | " | $OCF_2CHClF$ | |
| 453 | 2,6-$F_2$ | " | $OCF_2CHClF$ | |
| 454 | 2-Cl | " | $OCF_2CHFCF_3$ | |
| 455 | 2-F | " | $OCF_2CHFCF_3$ | |
| 456 | 2,6-$F_2$ | " | $OCF_2CHFCF_3$ | |
| 457 | 2-Cl | 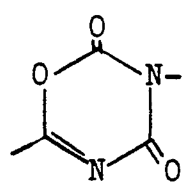 | $OCHF_2$ | |
| 458 | 2-F | | $OCHF_2$ | |
| 459 | 2,6-$F_2$ | | $OCHF_2$ | |
| 460 | 2-Cl | | $OCF_3$ | |
| 461 | 2-F | " | $OCF_3$ | |
| 462 | 2,6-$F_2$ | " | $OCF_3$ | |
| 463 | 2-Cl | " | $OCF_2CHF_2$ | |
| 464 | 2-F | " | $OCF_2CHF_2$ | |
| 465 | 2,6-$F_2$ | " | $OCF_2CHF_2$ | |

Fortsetzung der Tabelle 9

| Bsp. No. | $(Q^1)_r$ | G | $R^{10}$ | Fp (°C) |
|---|---|---|---|---|
| 466 | 2-Cl | 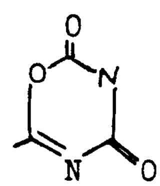 | $OCF_2CHFlF$ | |
| 467 | 2-F | | $OCF_2CHFlF$ | |
| 468 | 2,6-$F_2$ | | $OCF_2CHFlF$ | |
| 469 | 2-Cl | | $OCF_2CHFCF_3$ | |
| 470 | 2-F | ″ | $OCF_2CHFCF_3$ | |
| 471 | 2,6-$F_2$ | ″ | $OCF_2CHFCF_3$ | |

Tabelle 10

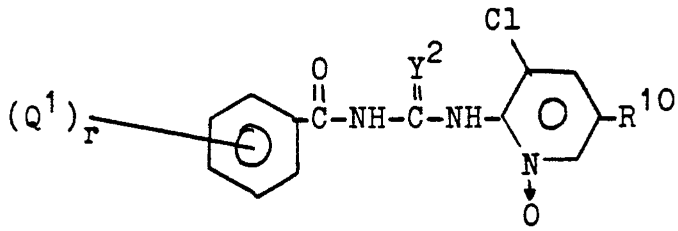

| Bsp. No. | $(Q^1)_r$ | $Y^2$ | $R^{10}$ | Fp (°C) |
|---|---|---|---|---|
| 472 | 2-Cl | O | $OCHF_2$ | |
| 473 | 2-F | O | $OCHF_2$ | |
| 474 | 2,6-$F_2$ | O | $OCHF_2$ | |
| 475 | 2-Cl | O | $OCF_3$ | |
| 476 | 2-F | O | $OCF_3$ | |
| 477 | 2,6-$F_2$ | O | $OCF_3$ | |
| 478 | 2-Cl | O | $OCF_2CHF_2$ | |
| 479 | 2-F | O | $OCF_2CHF_2$ | |
| 480 | 2,6-$F_2$ | O | $OCF_2CHF_2$ | |
| 481 | 2-Cl | O | $OCF_2CHClF$ | |
| 482 | 2-F | O | $OCF_2CHClF$ | |
| 483 | 2,6-$F_2$ | O | $OCF_2CHClF$ | |
| 484 | 2-Cl | O | $OCF_2CHFCF_3$ | |
| 485 | 2-F | O | $OCF_2CHFCF_3$ | |
| 486 | 2,6-$F_2$ | O | $OCF_2CHFCF_3$ | |
| 487 | 2-Cl | S | $OCHF_2$ | |
| 488 | 2-F | S | $OCHF_2$ | |
| 489 | 2,6-$F_2$ | S | $OCHF_2$ | |
| 490 | 2-Cl | S | $OCF_3$ | |
| 491 | 2-F | S | $OCF_3$ | |
| 492 | 2,6-$F_2$ | S | $OCF_3$ | |
| 493 | 2-Cl | S | $OCF_2CHF_2$ | |
| 494 | 2-F | S | $OCF_2CHF_2$ | |
| 495 | 2,6-$F_2$ | S | $OCF_2CHF_2$ | |

Fortsetzung der Tabelle 10

| Bsp. No. | $(Q^1)_r$ | $Y^2$ | $R^{10}$ | Fp (°C) |
|---|---|---|---|---|
| 496 | 2-Cl | S | $OCF_2CHClF$ | |
| 497 | 2-F | S | $OCF_2CHClF$ | |
| 498 | 2,6-$F_2$ | S | $OCF_2CHClF$ | |
| 499 | 2-Cl | S | $OCF_2CHFCF_3$ | |
| 500 | 2-F | S | $OCF_2CHFCF_3$ | |
| 501 | 2,6-$F_2$ | S | $OCF_2CHFCF_3$ | |

Biologische Beispiele

Beispiel I

Spodoptera-Test

Larven des afrikanischen Baumwollwurms (Spodoptera littoralis L III) und Petrischalen, in die eine Diät auf Agar-Basis eingefüllt wird, wurden in einer Spritzapparatur mit einer Wirkstoffzubereitung der gewünschten Konzentration behandelt. Die Larven wurden nach Antrocknen des Spritzbelages auf die Agar-Diät gesetzt.

Nach der gewünschten Zeit (L III bis Falterschlupf) wurde die Abtötung der Raupen bzw. der Schlupf der Falter in % bestimmt. Dabei bedeutet 100 %, daß alle Raupen abgetötet wurden bzw. kein Falter aus den Raupen schlüpfte.

Bei diesem Test zeigten bei 100 ppm Wirkstoff in der Spritzbrühe 100 % Wirksamkeit die Verbindungen der Beispiele 1, 2, 82, 83, 90, 104, 105, 145 und 147.

Beispiel II

Musca-Test

24 Stunden alte Hausfliegenlarven (Musca domestica) wurden in eine Fliegendiät, die vorher mit einer Wirkstoffzubereitung der gewünschten Konzentration behandelt wurde, eingebracht.

Nach der gewünschten Zeit (L I bis Fliegenschlupf) wurde die Abtötung der Larven bzw. der Schlupf der Fliegen in % bestimmt. Dabei bedeutet 100 %, daß alle Larven abgetötet wurden bzw. keine Fliege aus den Puppen schlüpfte.

Bei 100 ppm Wirkstoff erreichten 100 % Wirksamkeit die Verbindungen der Beispiele 1, 2, 82, 83, 90, 104 und 105.

Beispiel III

Aedes-Test

Man füllte die wäßrigen Wirkstoffzubereitungen der gewünschten Konzentration in Erlenmeyerkolben und setzte anschließend 24 Stunden alte Gelbfiebermückenlarven (Aedes ägypti) in die Kolben.

Nach der gewünschten Zeit (bis zum Mückenschlupf) wurde die Abtötung der Larven bzw. der Schlupf der Mücken in % bestimmt. Dabei bedeutet 100 %, daß alle Larven abgetötet wurden bzw. keine Mücke - schlüpfte.

Bei 100 ppm Wirkstoff erreichten 100 % Wirksamkeit die Verbindungen der Beispiele 1, 2, 82 und 104.

Beispiel IV

Oncopeltus-Test

Larven einer Baumwollwanze (Oncopeltus fasciatus L III) wurden zusammen mit einem Dentalröllchen, das vorher mit einer Wirkstoffzubereitung der gewünschten Konzentration behandelt wurde, in einen Plastikbecher gegeben.

Nach der gewünschten Zeit (L III bis Imago) wurde die Abtötung der Larven bzw. der Schlupf der Imago in % bestimmt. Dabei bedeutet 100 %, daß alle Larven abgetötet wurden bzw. keine Imago aus dem letzten Larvenstadium schlüpfte.

Bei 100 ppm Wirkstoff erreichten 100 % Wirksamkeit die Verbindungen gemäß Beispiel 1, 2, 82, 90 und 104.

Beispiel V

Heliothis-Test

Larven der amerikanischen Tabakeule (Heliothis virescens, L III) und Petrischalen, in die eine Diät auf Agar-Basis eingefüllt wird, wurden in einer Spritzapparatur mit einer Wirkstoffzubereitung der gewünschten Konzentration behandelt. Die Larven wurden nach Antrocknen des Spritzbelages auf die Agar-Dät gesetzt.

Nach der gewünschten Zeit (7 Tage, Häutung L III nach L IV) wurde die Abtötung der Raupen in % bestimmt. Dabei bedeutet 100 %, daß alle Raupen abgetötet wurden.

Bei 100 ppm Wirkstoff erreichten 100 % Wirksamkeit die Verbindungen gemäß Beispiel 1, 2, 82, 83, 90, 104 und 105.

Beispiel VI

Trialeurodes-Test

Mit weißer Fliege (Trialeurodes Vaporariorum, Eier) stark besetzte Bohnenpflanzen (Phaseolus vulgaris) wurden mit Wirkstoff der gewünschten Konzentration in der Spritzbrühe bis zum beginnenden Abtropfen gespritzt. Nach Aufstellung der Pflanzen im Gewächshaus bei 20-25°C und 40-50 % relative Feuchte erfolgte nach 14 Tagen die mikroskopische Kontrolle.

Bei 20 ppm Wirkstoff erreichten 100 % Wirksamkeit die Verbindungen der Beispiele 1, 2, 82, 83, 90 und 105.

**Ansprüche**

1. Verbindungen der Formel I,

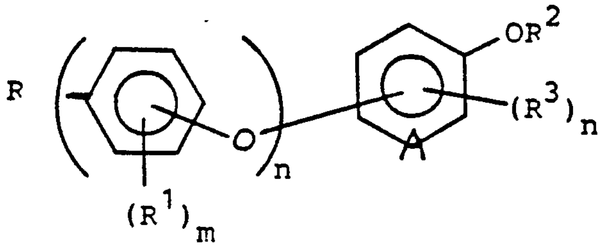

(I),

worin

A = N oder N → O,

R = einen Rest der Formeln

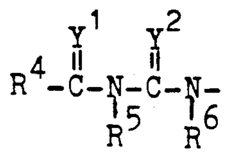

,

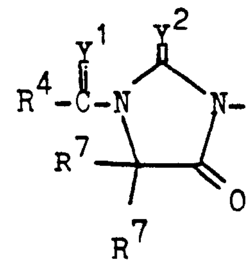

,

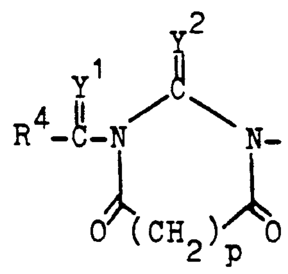

,

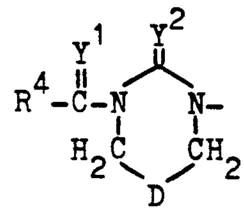

,

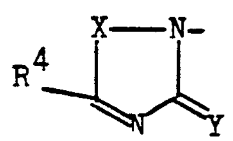

,

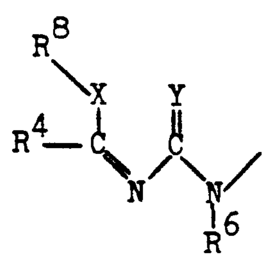

,

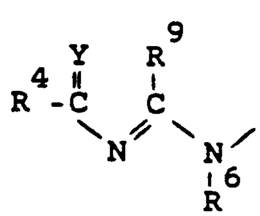

,

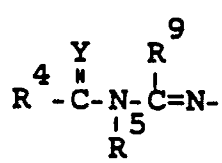

oder

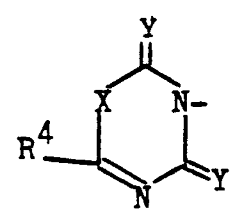

,

$R^1$ jeweils unabhängig voneinander Halogen, ($C_1$-$C_6$)-Alkyl, ($C_2$-$C_6$)-Alkenyl, ($C_2$-$C_6$)-Alkinyl, ($C_1$-$C_6$)-Alkoxy, ($C_1$-$C_6$)-Alkoxy-carbonyl, ($C_1$-$C_6$-Alkyl)-carbonyl, wobei die Reste ein-oder mehrfach durch Halogen substituiert sein können, Nitro, Cyano oder Carboxy,

$R^2$ jeweils unabhängig voneinander halogeniertes ($C_1$-$C_6$)-Alkyl, halogeniertes ($C_2$-$C_6$)-Alkenyl oder halogeniertes ($C_2$-$C_6$)-Alkinyl,

$R^3$ unabhängig voneinander Halogen,

$R^4$ Phenyl, das durch Halogen, ($C_1$-$C_3$)-Alkyl, ($C_1$-$C_3$)-Alkoxy, Halo($C_1$-$C_3$)-alkyl oder Halo($C_1$-$C_3$)-alkoxy substituiert sein kann,

$R^5$ Wasserstoff, ($C_1$-$C_6$)-Alkoxy, ($C_1$-$C_6$)Alkylthio, Benzyl, halogeniertes ($C_1$-$C_6$)-Alkoxy, halogeniertes ($C_1$-$C_6$)-Alkylthio oder halogeniertes Benzyl,

$R^6$ H, ($C_1$-$C_6$)-Alkyl, ($C_1$-$C_6$)Alkylthio, ($C_1$-$C_6$)-Alkylsufonyl, die alle halogeniert sein können, Phenylthio oder Phenylsulfonyl, das durch ($C_1$-$C_6$)-Alkyl, ($C_1$-$C_6$)-Alkoxy, ($C_1$-$C_6$)-Alkoxycarbonyl, halogeniertes ($C_1$-$C_6$)-Alkyl,

halogeniertes $(C_1\text{-}C_6)$-alkoxy, halogeniertes $(C_1\text{-}C_4)$-Alkoxycarbonyl, Halogen, Nitro oder Cyano substituiert ist, Phosphoryl, Thiophosphoryl, die beide durch zwei Reste der Gruppe $(C_1\text{-}C_6)$-Alkoxy, $(C_1\text{-}C_6)$-Alkylthio, Amino, $(C_1\text{-}C_6)$-Alkylamino oder Di-$(C_1\text{-}C_6$-Alkyl)-amino substituiert sind,

$R^7$ unabhängig voneinander H, Halogen oder $(C_1\text{-}C_3)$-Alkyl,

$R^8$ $(C_1\text{-}C_8)$-Alkyl, $(C_2\text{-}C_8)$-Alkenyl, $(C_2\text{-}C_8)$-Alkinyl, Phenyl oder Benzyl, die alle halogeniert sein können,

$R^9$ einen Rest der Formeln $-XR^8$, $-S\text{-}S\text{-}R^{10}$, Benzimidazolyl, Benztriazolyl, Pyrazolyl, wobei diese drei Reste durch $(C_1\text{-}C_3)$-Alkyl oder Halogen substituiert sein können, Triazolyl oder Imidazolyl, die beide durch $(C_1\text{-}C_3)$-Alkyl substituiert sein können,

$R^{10}$ $(C_1\text{-}C_{18})$-Alkyl, $(C_3\text{-}C_8)$-Cycloalkyl, Phenyl-$(C_1\text{-}C_4)$-alkyl oder Phenyl, die alle durch Halogen, Nitro oder $(C_1\text{-}C_8)$-Alkyl substituiert sein können,

D gegebenenfalls verzweigtes $C_1\text{-}C_2$-Alkylen, O oder S,

X, Y, $Y^1$, $Y^2$ unabhängig voneinander O oder S,

n unabhängig voneinander 0, 1 oder 2,

m 0, 1, 2 oder 3 und

p 0 oder 1 bedeuten, sowie deren für die Landwirtschaft einsetzbaren Salze.

2. Verbindungen der Formel I von Anspruch 1 worin

A = N

R = einen Rest der Formeln

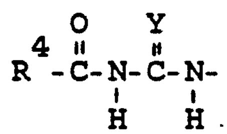

,

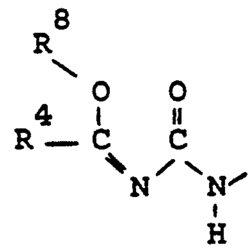

,

oder

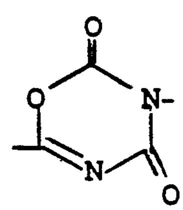

$R^1$ jeweils unabhängig voneinander Halogen, $(C_1\text{-}C_6)$-Alkyl oder $(C_1\text{-}C_6)$Alkoxy-carbonyl, wobei die Reste ein- oder mehrfach durch Halogen substituiert sein können,

$R^2$ jeweils unabhängig voneinander halogeniertes $(C_1\text{-}C_6)$-Alkyl, halogeniertes $(C_2\text{-}C_6)$-Alkenyl oder halogeniertes $(C_2\text{-}C_6)$-Alkinyl,

$R^3$ unabhängig voneinander Halogen,

$R^4$ Phenyl, das durch Halogen, $(C_1\text{-}C_3)$-Alkyl, $(C_1\text{-}C_3)$-Alkoxy, Halo$(C_1\text{-}C_3)$-alkyl oder Halo$(C_1\text{-}C_3)$-alkoxy substituiert sein kann,

$R^8$ $(C_1\text{-}C_6)$Alkyl, das halogeniert sein kann,

X unabhängig voneinander O oder S,

n im Falle des Phenoxyrestes 0 oder 1 und im Falle des Restes $R^3$ 0, 1 oder 2 und

m 0, 1, 2 oder 3 bedeuten.

3. Verbindungen der Formel I von Anspruch 2, worin

$R^1$ Halogen in den Positionen 2, 3 oder 5 des Phenoxyringes relativ zur Stellung von R, Halo $(C_1\text{-}C_6)$alkyl oder $(C_1\text{-}C_3)$Alkoxycarbonyl jeweils in den Positionen 3 oder 5 des Phenoxyringes

$R^2$ $(C_1\text{-}C_3)$-Haloalkoxy

$R^3$ F, Cl oder Br und

$R^4$ Phenyl bedeutet, das durch Halogen, insbesondere in den Positionen 2 oder 6 substituiert ist,

und A, $R^8$, X, n und m die in Anspruch 2 angegebenen Bedeutungen besitzen.

4. Verfahren zur Herstellung der Verbindungen der Formel I, von Ansprüchen 1 bis 3, dadurch gekennzeichnet, daß man

a) fürR = $R^4\text{-}CY^1\text{-}NR^5\text{-}CY^2\text{-}NR^6\text{-}$

a1) eine Verbindung der Formel (II) mit

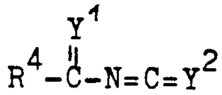 (II)

einer Verbindung der Formel III

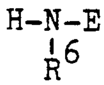 (III), worin E = 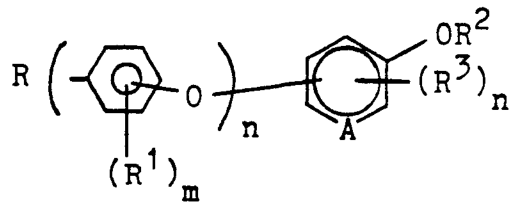

bedeutet, umsetzt, oder

a2) eine Verbindung der Formel IV

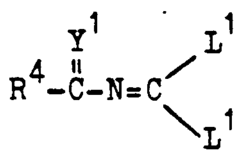 (IV),

worin $L^1$ unabhängig voneinander Halogen, $(C_1\text{-}C_3)$-Alkylthio, $(C_1\text{-}C_3)$-Alkylsulfenyl oder $(C_1\text{-}C_3)$-Alkylsulfonyl bedeutet,

mit einer Verbindung der Formel III und anschließend mit einer Verbindung der Formel $H_2Y$ umsetzt, oder

a3) eine Verbindung der Formel V

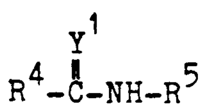 (V),

mit einer Verbindung der Formel VIa

$Y^2 = C = N\text{-}E$ (VIa),

oder

a4) eine Verbindung der Formel V mit einer Verbindung der Formel VIb

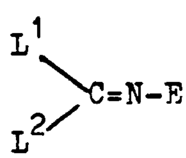 (VIb),

wobei $L^2$ die Bedeutung von $L^1$ besitzt und zusätzlich für Mercapto steht, und anschließend mit einer Verbindung der Formel $H_2Y$ umsetzt, oder

a5) eine Verbindung der Formel V mit einer Verbindung der Formel VII

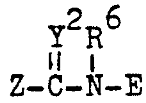 (VII),

worin Z = ($C_1$-$C_6$)-Alkoxy, ($C_1$-$C_6$)-Alkylthio, Benzyloxy, die alle halogeniert sein können, Triazolyl oder Imidazolyl oder einen Rest der Formel -$NHR^5$ beeutet, umsetzt, oder

b) für R = 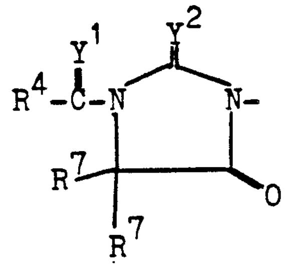

eine Verbindung der Formel VIII

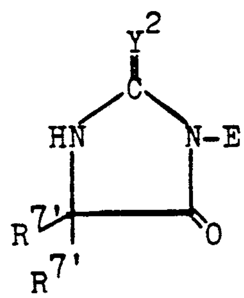

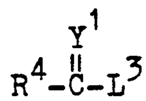

(VIII) (IX),

wobei $R^{7'}$ = H oder ($C_1$-$C_3$)-Alkyl bedeutet, mit einer Verbindung der Formel IX, wobei $L^3$ = eine Abgangsgruppe wie Halogen oder Alkylthio bedeutet, umsetzt und die erhaltenen Verbindungen gegebenfalls halogeniert oder alkyliert, oder

c) für R = 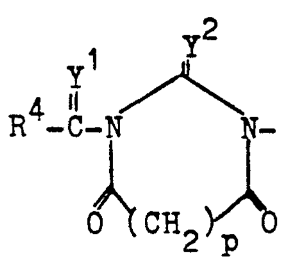 oder 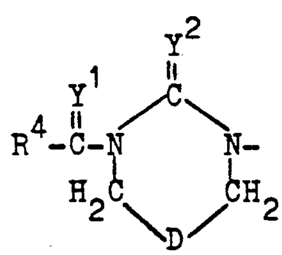

eine gemäß Verfahren a) erhaltene Verbindung der Formel I mit $R^5$, $R^6$ = H mit einer Verbindung Xa oder Xb, worin $L^4$ = eine Abgangsgruppe wie Halogen bedeutet,

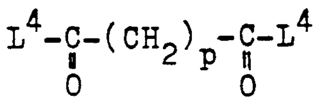

$L^4-CH_2-D-CH_2-L^4$

Xa Xb

umsetzt, oder

d) für R = 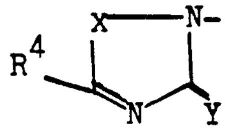

d1) für X = O eine Verbindung der Formel XI mit

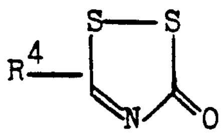 (XI) HONH-E (XII)

einer Verbindung der Formel XII umsetzt und die erhaltene Verbindung der Formel I mit Y = O auf bekannte Weise thioliert, oder

d2) für X = S eine gemäß a) erhaltene Verbindung der Formel I mit Y' = S und $R^5$, $R^6$ = H cyclisiert, oder

e) für R = 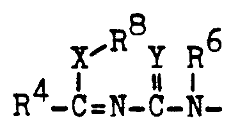

e1) eine Verbindung der Formel XIII

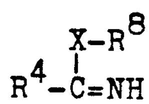

(XIII)

mit einer Verbindung der Formel VIa oder mit einer Verbindung der Formel

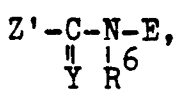

worin Z' die Bedeutung von Z außer $-NHR^5$ besitzt, umsetzt, oder

e2) eine Verbindung der Formel XIV

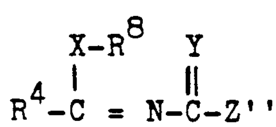 (XIV),

worin Z" die Bedeutung von Z' besitzt oder zusätzlich für Halogen steht, mit einer Verbindung der Formel III umsetzt, oder

f) für R = 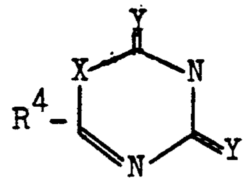

eine Verbindung der Formel II mit einer Verbindung der Formel VIa umsetzt, oder

g) für R = 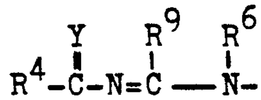

g1) eine Verbindung der Formel XV

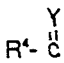 (XV)

mit einer Verbindung der Formel XVI

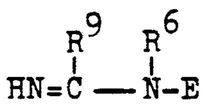 (XVI) umsetzt, oder

g2) eine Verbindung der Formel XVII

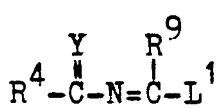 (XVII)

mit einer Verbindung der Formel III umsetzt, oder

g3) für $R^9$ = ein obengenannter Rest außer $-X-R^8$ eine unter a) erhaltene Verbindung der Formel (I) mit $Y^2$ = S und $R^5$, $R^6$ = H mit einer Verbindung der Formel $R^{9'}-SO-R^{9'}$ wobei $R^{9'}$ die Bedeutung von $R^9$ außer $-XR^8$ und $-S-S-R^{10}$ besitzt, oder mit einer Verbindung der Formel $L^5-S-S-R^{10}$, wobei $L^5$ = Halogen, N-Succinyl oder ein analoger Rest bedeutet, umsetzt, oder

h) für R = 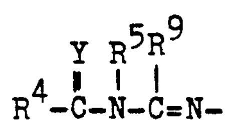

eine Verbindung der Formel XV mit einer Verbindung der Formel XVIII

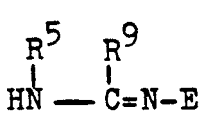 (XVIII) umsetzt.

5. Insektizide, akarizide, nematozide und molluskizide Mittel, dadurch gekennzeichnet, daß sie eine wirksame Menge einer Verbindung der Formel I enthalten.

6. Verwendung der Verbindungen der Formel I zur Bekämpfung von Schadinsekten, Akariden, Nematoden und Mollusken.

7. Verfahren zur Bekämpfung von Schadinsekten, Akariden, Nematoden und Mollusken, dadurch gekennzeichnet, daß man auf diese oder die von Ihnen befallenen Pflanzen, Flächen oder Substrate eine wirksame Menge einer Verbindung der Formel I appliziert.

<u>Patentansprüche für folgenden Vertragstaat: AT:</u>

1. Verfahren zur Herstellung der Verbindungen der Formel I,

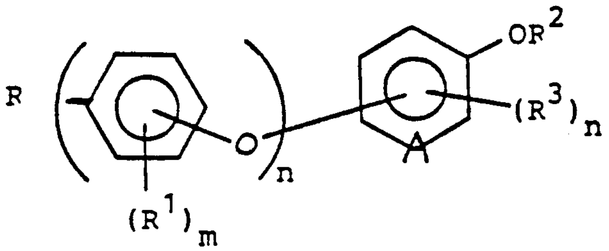

(I),

worin
A = N oder N → O,
R = einen Rest der Formeln

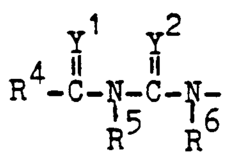

,

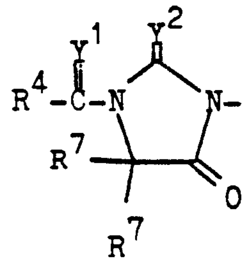

,

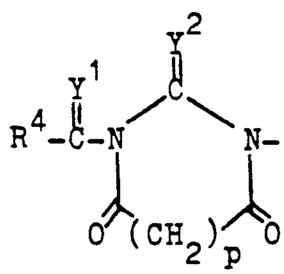

,

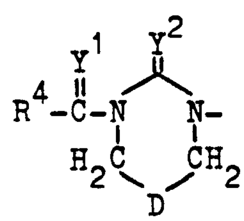

,

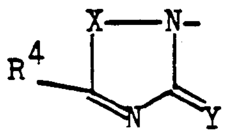

,

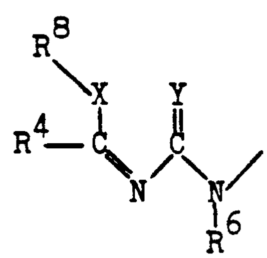

,

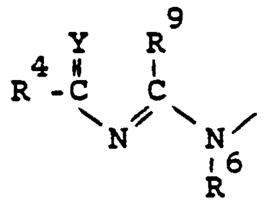 , 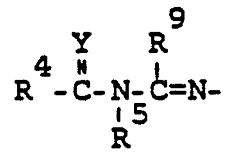 oder 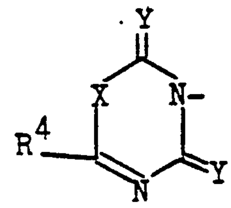 ,

$R^1$ jeweils unabhängig voneinander Halogen, ($C_1$-$C_6$)-Alkyl, ($C_2$-$C_6$)-Alkenyl, ($C_2$-$C_6$)-Alkinyl, ($C_1$-$C_6$)-Alkoxy, ($C_1$-$C_6$)-Alkoxy-carbonyl, ($C_1$-$C_6$-Alkyl)-carbonyl, wobei die Reste ein-oder mehrfach durch Halogen substituiert sein können, Nitro, Cyano oder Carboxy,

$R^2$ jeweils unabhängig voneinander halogeniertes ($C_1$-$C_6$)-Alkyl, halogeniertes ($C_2$-$C_6$)-Alkenyl oder halogeniertes ($C_2$-$C_6$)-Alkinyl,

$R^3$ unabhängig voneinander Halogen,

$R^4$ Phenyl, das durch Halogen, ($C_1$-$C_3$)-Alkyl, ($C_1$-$C_3$)-Alkoxy, Halo($C_1$-$C_3$)-alkyl oder Halo($C_1$-$C_3$)-alkoxy substituiert sein kann,

$R^5$ Wasserstoff, ($C_1$-$C_6$)-Alkoxy, ($C_1$-$C_6$)Alkylthio, Benzyl, halogeniertes ($C_1$-$C_6$)-Alkoxy, halogeniertes ($C_1$-$C_6$)-Alkylthio oder halogeniertes Benzyl,

$R^6$ H, ($C_1$-$C_6$)-Alkyl, ($C_1$-$C_6$)Alkylthio, ($C_1$-$C_6$)-Alkylsulfonyl, die alle halogeniert sein können, Phenylthio oder Phenylsulfonyl, das durch ($C_1$-$C_6$)-Alkyl, ($C_1$-$C_6$)-Alkoxy, ($C_1$-$C_6$)-Alkoxycarbonyl, halogeniertes ($C_1$-$C_6$)-Alkyl, halogeniertes ($C_1$-$C_6$)-alkoxy, halogeniertes ($C_1$-$C_4$)-Alkoxycarbonyl, Halogen, Nitro oder Cyano substituiert ist, Phosphoryl, Thiophosphoryl, die beide durch zwei Reste der Gruppe ($C_1$-$C_6$)-Alkoxy, ($C_1$-$C_6$)-Alkylthio, Amino, ($C_1$-$C_6$)-Alkylamino oder Di-($C_1$-$C_6$-Alkyl)-amino substituiert sind,

$R^7$ unabhängig voneinander H, Halogen oder ($C_1$-$C_3$)-Alkyl,

$R^8$ ($C_1$-$C_8$)-Alkyl, ($C_2$-$C_8$)-Alkenyl, ($C_2$-$C_8$)-Alkinyl, Phenyl oder Benzyl, die alle halogeniert sein können,

$R^9$ einen Rest der Formeln -$XR^8$, -S-S-$R^{10}$, Benzimidazolyl, Benztriazolyl, Pyrazolyl, wobei diese drei Reste durch ($C_1$-$C_3$)-Alkyl oder Halogen substituiert sein können, Triazolyl oder Imidazolyl, die beide durch ($C_1$-$C_3$)-Alkyl substituiert sein können,

$R^{10}$ ($C_1$-$C_{18}$)-Alkyl, ($C_3$-$C_8$)-Cycloalkyl, Phenyl-($C_1$-$C_4$)-alkyl oder Phenyl, die alle durch Halogen, Nitro oder ($C_1$-$C_8$)-Alkyl substituiert sein können,

D gegebenenfalls verzweigtes $C_1$-$C_2$-Alkylen, O oder S,

X, Y, $Y^1$, $Y^2$ unabhängig voneinander O oder S,

n unabhängig voneinander 0, 1 oder 2,

m 0, 1, 2 oder 3 und

p 0 oder 1 bedeuten, sowie deren für die Landwirtschaft einsetzbaren Salze, dadurch gekennzeichnet, daß man

a) für R = $R^4$-$CY^1$-$NR^5$-$CY^2$-$NR^6$-

a1) eine Verbindung der Formel (II) mit

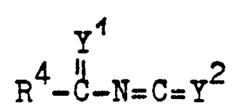 (II)

einer Verbindung der Formel III

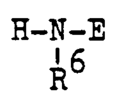 (III), worin E = 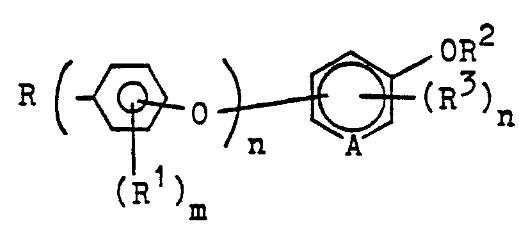

bedeutet, umsetzt, oder

a2) eine Verbindung der Formel IV

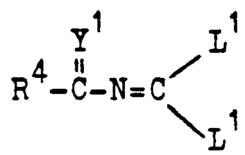 (IV),

worin $L^1$ unabhängig voneinander Halogen, $(C_1$-$C_3)$-Alkylthio, $(C_1$-$C_3)$-Alkylsulfenyl oder $(C_1$-$C_3)$-Alkylsulfonyl bedeutet,
mit einer Verbindung der Formel III und anschließend mit einer Verbindung der Formel $H_2Y$ umsetzt, oder
a3) eine Verbindung der Formel V

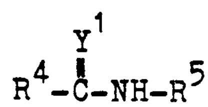 (V),

mit einer Verbindung der Formel VIa
$Y^2 = C = N$-E (VIa),
oder
a4) eine Verbindung der Formel V mit einer Verbindung der Formel VIb

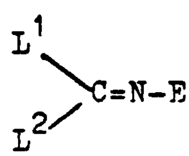 (VIb),

wobei $L^2$ die Bedeutung von $L^1$ besitzt und zusätzlich für Mercapto steht,
und anschließend mit einer Verbindung der Formel $H_2Y$ umsetzt, oder
a5) eine Verbindung der Formel V mit einer Verbindung der Formel VII

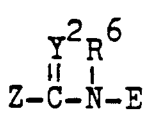 (VII),

worin Z = $(C_1$-$C_6)$-Alkoxy, $(C_1$-$C_6)$-Alkylthio, Benzyloxy, die alle halogeniert sein können, Triazolyl oder Imidazolyl oder ienen Rest der Formel -$NHR^5$ beeutet, umsetzt, oder

b) für R = 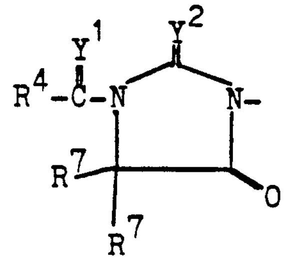

eine Verbindung der Formel VIII

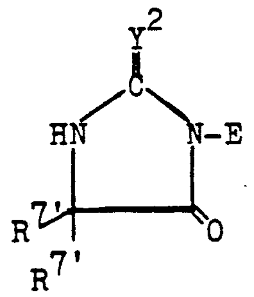

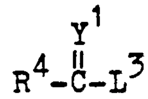

(VIII) (IX),

wobei $R^{7'}$ = H oder ($C_1$-$C_3$)-Alkyl bedeutet, mit einer Verbindung der Formel IX, wobei $L^3$ = eine Abgangsgruppe wie Halogen oder Alkylthio bedeutet, umsetzt und die erhaltenen Verbindungen gegebenenfalls halogeniert oder alkyliert, oder

c) für R = 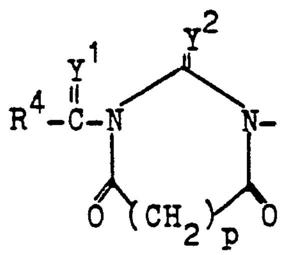 oder 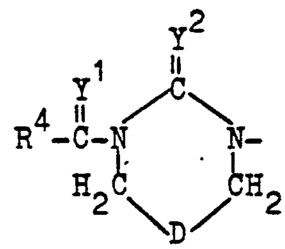

eine gemäß Verfahren a) erhaltene Verbindung der Formel I mit $R^5$, $R^6$ = H mit einer Verbindung Xa oder Xb, worin $L^4$ = eine Abgangsgruppe wie Halogen bedeutet,

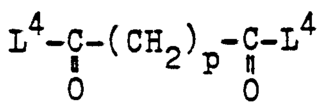

$L^4-CH_2-D-CH_2-L^4$

Xa Xb

umsetzt, oder

d) für R = 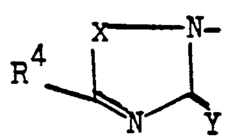

d1) für X = O eine Verbindung der Formel XI mit

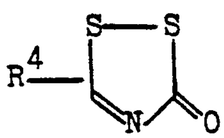 (XI) HONH-E (XII)

einer Verbindung der Formel XII umsetzt und die erhaltene Verbindung der Formel I mit Y = O auf bekannte Weise thioliert, oder

d2) für X = S eine gemäß a) erhaltene Verbindung der Formel I mit $Y^1$ = S und $R^5$, $R^6$ = H cyclisiert, oder

d) für R = 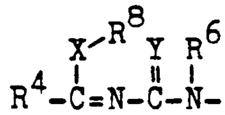

e1) eine Verbindung der Formel XIII

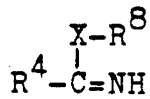

(XIII)

mit einer Verbindung der Formel VIa oder mit einer Verbindung der Formel

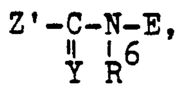

worin Z' die Bedeutung von Z außer $-NHR^5$ besitzt, umsetzt, oder

e2) eine Verbindung der Formel XIV

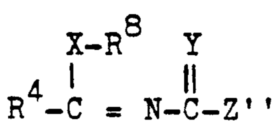 (XIV),

worin Z" die Bedeutung von Z' besitzt oder zusätzlich für Halogen steht, mit einer Verbindung der Formel III umsetzt, oder

f) für R = 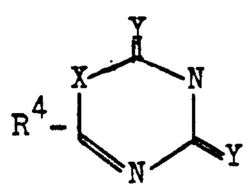

eine Verbindung der Formel II mit einer Verbindung der Formel VIa umsetzt, oder

g) für R = 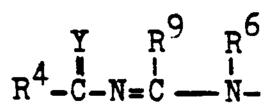

g1) eine Verbindung der Formel XV

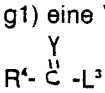 (XV)

mit einer Verbindung der Formel XVI

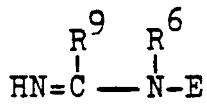 (XVI) umsetzt, oder

g2) eine Verbindung der Formel XVII

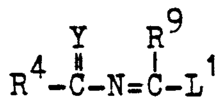 (XVII)

mit einer Verbindung der Formel III umsetzt, oder
g3) für $R^9$ = ein obengenannter Rest außer $-X-R^8$ eine unter a) erhaltene Verbindung der Formel (I) mit $Y^2$ = S und $R^5$, $R^6$ = H mit einer Verbindung der Formel $R^{9'}$-SO-$R^{9'}$ wobei $R^{9'}$ die Bedeutung von $R^9$ außer $-XR^8$ und $-S-S-R^{10}$ besitzt, oder mit einer Verbindung der Formel $L^5$-S-S-$R^{10}$, wobei $L^5$ = Halogen, N-Succinyl oder ein analoger Rest bedeutet, umsetzt, oder

h) für R = 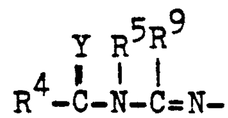

eine Verbindung der Formel XV mit einer Verbindung der Formel XVIII

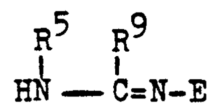 (XVIII)

umsetzt und die erhaltene Verbindung gegebenenfalls in ihr Salz überführt.

2. Verfahren gemäß Anspruch 1, dadurch gekennzeichnet, daß
A = N
R = einen Rest der Formeln

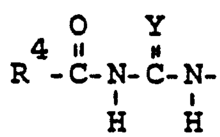 , 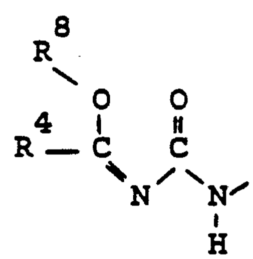 ,

oder 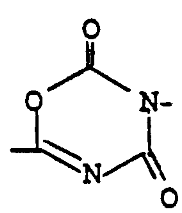

$R^1$ jeweils unabhängig voneinander Halogen, $(C_1-C_6)$-Alkyl oder $(C_1-C_6)$Alkoxy-carbonyl, wobei die Reste ein- oder mehrfach durch Halogen substituiert sein können,
$R^2$ jeweils unabhängig voneinander halogeniertes $(C_1-C_6)$-Alkyl, halogeniertes $(C_2-C_6)$-Alkenyl oder halogeniertes $(C_2-C_6)$-Alkinyl,
$R^3$ unabhängig voneinander Halogen,
$R^4$ Phenyl, das durch Halogen, $(C_1-C_3)$-Alkyl, $(C_1-C_3)$-Alkoxy, Halo$(C_1-C_3)$-alkyl oder Halo$(C_1-C_3)$-alkoxy substituiert sein kann,
$R^8$ $(C_1-C_6)$Alkyl, das halogeniert sein kann,
X unabhängig voneinander O oder S,
n im Falle des Phenoxyrestes 0 oder 1 und im Falle des Restes $R^3$ 0, 1 oder 2 und
m 0, 1, 2 oder 3 bedeuten.

3. Verfahren gemäß Anspruchen 2 oder 3, dadurch gekennzeichnet, daß
$R^1$ Halogen in den Positionen 2, 3 oder 5 des Phenoxyringes relativ zur Stellung von R, Halo $(C_1-C_6)$alkyl oder $(C_1-C_3)$Alkoxycarbonyl jeweils in den Positionen 3 oder 5 des Phenoxyringes
$R^2$ $(C_1-C_3)$-Haloalkoxy
$R^3$ F, Cl oder Br und
$R^4$ Phenyl bedeutet, das durch Halogen, insbesondere in den Positionen 2 oder 6 substituiert ist,
und A, $R^8$, X, n und m die in Anspruch 2 angegebenen Bedeutungen besitzen.

4. Insektizide, akarizide, nematozide und molluskizide Mittel, dadurch gekennzeichnet, daß sie eine wirksame Menge einer Verbindung der Formel I enthalten.

5. Verwendung der Verbindungen der Formel I zur Bekämpfung von Schadinsekten, Akariden, Nematoden und Mollusken.

6. Verfahren zur Bekämpfung von Schadinsekten, Akariden, Nematoden und Mollusken, dadurch gekennzeichnet, daß man auf diese oder die von Ihnen befallenen Pflanzen, Flächen oder Substrate eine wirksame Menge einer Verbindung der Formel I appliziert.

<u>Patentansprüche für folgenden Vertragstaat: ES:</u>

1. Verfahren zur Herstellung der Verbindungen der Formel I,

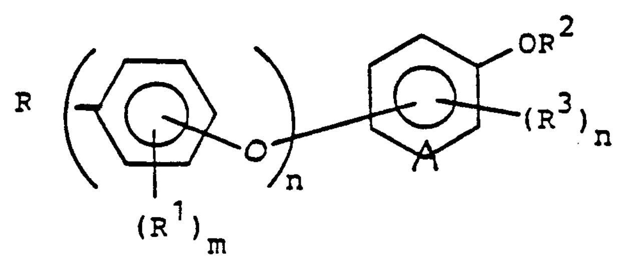

(I),

worin
A = N oder N → O,
R = einen Rest der Formeln

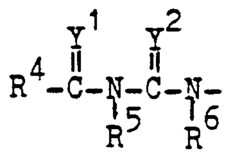 , 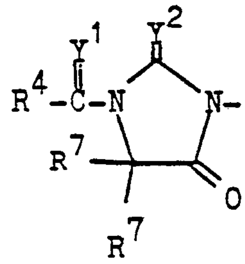 ,

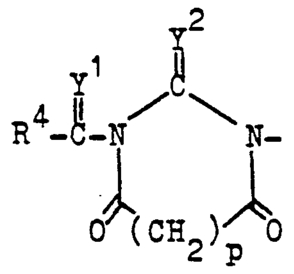 , 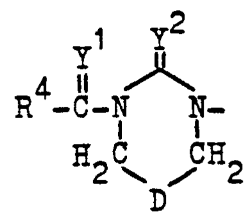 ,

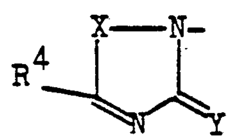 , 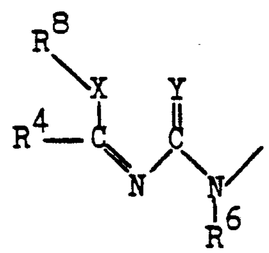 ,

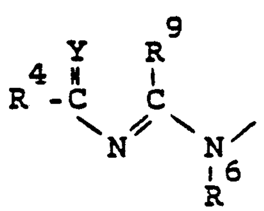 , 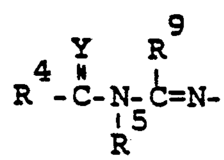 oder 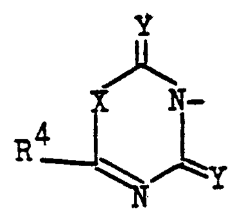 ,

$R^1$ jeweils unabhängig voneinander Halogen, $(C_1-C_6)$-Alkyl, $(C_2-C_6)$-Alkenyl, $(C_2-C_6)$-Alkinyl, $(C_1-C_6)$-Alkoxy, $(C_1-C_6)$-Alkoxy-carbonyl, $(C_1-C_6$-Alkyl)-carbonyl, wobei die Reste ein-oder mehrfach durch Halogen substituiert sein können, Nitro, Cyano oder Carboxy,

$R^2$ jeweils unabhängig voneinander halogeniertes $(C_1-C_6)$-Alkyl, halogeniertes $(C_2-C_6)$-Alkenyl oder halogeniertes $(C_2-C_6)$-Alkinyl,

$R^3$ unabhängig voneinander Halogen,

$R^4$ Phenyl, das durch Halogen, $(C_1-C_3)$-Alkyl, $(C_1-C_3)$-Alkoxy, Halo$(C_1-C_3)$-alkyl oder Halo$(C_1-C_3)$-alkoxy substituiert sein kann,

$R^5$ Wasserstoff, $(C_1-C_6)$-Alkoxy, $(C_1-C_6)$Alkylthio, Benzyl, halogeniertes $(C_1-C_6)$-Alkoxy, halogeniertes $(C_1-C_6)$-Alkylthio oder halogeniertes Benzyl,

$R^6$ H, $(C_1-C_6)$-Alkyl, $(C_1-C_6)$Alkylthio, $(C_1-C_6)$-Alkylsulfonyl, die alle halogeniert sein können, Phenylthio oder Phenylsulfonyl, das durch $(C_1-C_6)$-Alkyl, $(C_1-C_6)$-Alkoxy, $(C_1-C_6)$-Alkoxycarbonyl, halogeniertes $(C_1-C_6)$-Alkyl, halogeniertes $(C_1-C_6)$-alkoxy, halogeniertes $(C_1-C_4)$-Alkoxycarbonyl, Halogen, Nitro oder Cyano substituiert ist, Phosphoryl, Thiophosphoryl, die beide durch zwei Reste der Gruppe $(C_1-C_6)$-Alkoxy, $(C_1-C_6)$-Alkylthio, Amino, $(C_1-C_6)$-Alkylamino oder Di-$(C_1-C_6$-Alkyl)-amino substituiert sind,

$R^7$ unabhängig voneinander H, Halogen oder $(C_1-C_3)$-Alkyl,

$R^8$ $(C_1-C_8)$-Alkyl, $(C_2-C_8)$-Alkenyl, $(C_2-C_8)$-Alkinyl, Phenyl oder Benzyl, die alle halogeniert sein können,

$R^9$ einen Rest der Formeln $-XR^8$, $-S-S-R^{10}$, Benzimidazolyl, Benztriazolyl, Pyrazolyl, wobei diese drei Reste

durch $(C_1-C_3)$-Alkyl oder Halogen substituiert sein können, Triazolyl oder Imidazolyl, die beide durch $(C_1-C_3)$-Alkyl substituiert sein können,
$R^{10}$ $(C_1-C_{18})$-Alkyl, $(C_3-C_8)$-Cycloalkyl, Phenyl-$(C_1-C_4)$-alkyl oder Phenyl, die alle durch Halogen, Nitro oder $(C_1-C_8)$-Alkyl substituiert sein können,
D gegebenenfalls verzweigtes $C_1-C_2$-Alkylen, O oder S,
X, Y, $Y^1$, $Y^2$ unabhängig voneinander O oder S,
n unabhängig voneinander 0, 1 oder 2,
m 0, 1, 2 oder 3 und
p 0 oder 1 bedeuten, sowie deren für die Landwirtschaft einsetzbaren Salze, dadurch gekennzeichnet, daß man
a) für R = $R^4$-$CY^1$-$NR^5$-$CY^2$-$NR^6$-
a1) eine Verbindung der Formel (II) mit

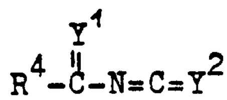 (II)

einer Verbindung der Formel III

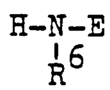 (III), worin E = 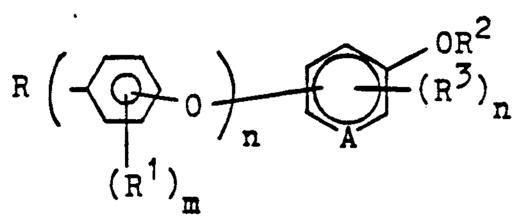

bedeutet, umsetzt, oder
a2) eine Verbindung der Formel IV

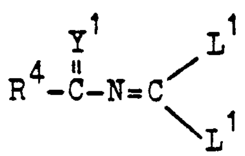 (IV),

worin $L^1$ unabhängig voneinander Halogen, $(C_1-C_3)$-Alkylthio, $(C_1-C_3)$-Alkylsulfenyl oder $(C_1-C_3)$-Alkylsulfonyl bedeutet,
mit einer Verbindung der Formel III und anschließend mit einer Verbindung der Formel $H_2Y$ umsetzt, oder
a3) eine Verbindung der Formel V

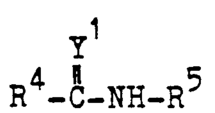 (V),

mit einer Verbindung der Formel VIa
$Y^2=C=N-E$ (VIa),
oder
a4) eine Verbindung der Formel V mit einer Verbindung der Formel VIb

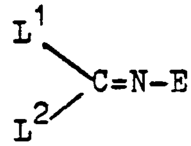 (VIb),

wobei $L^2$ die Bedeutung von $L^1$ besitzt und zusätzlich für Mercapto steht, und anschließend mit einer Verbindung der Formel $H_2Y$ umsetzt, oder
a5) eine Verbindung der Formel V mit einer Verbindung der Formel VII

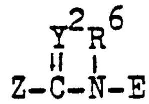 (VII),

worin Z = ($C_1$-$C_6$)-Alkoxy, ($C_1$-$C_6$)-Alkylthio, Benzyloxy, die alle halogeniert sein können, Triazolyl oder Imidazolyl oder ienen Rest der Formel -$NHR^5$ beeutet, umsetzt, oder

b) für R = 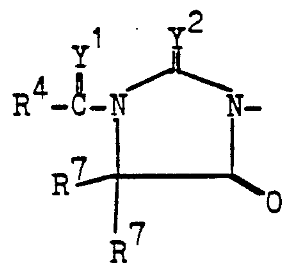

eine Verbindung der Formel VIII

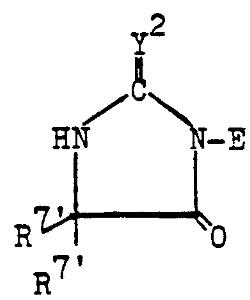 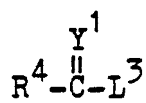

(VIII) (IX),

wobei $R^{7'}$ = H oder ($C_1$-$C_3$)-Alkyl bedeutet, mit einer Verbindung der Formel IX, wobei $L^3$ = eine Abgangsgruppe wie Halogen oder Alkylthio bedeutet, umsetzt und die erhaltenen Verbindungen gegebenfalls halogeniert oder alkyliert, oder

c) für R = 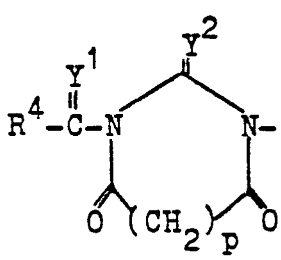 oder 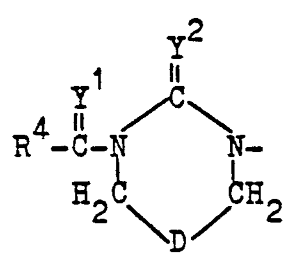

eine gemäß Verfahren a) erhaltene Verbindung der Formel I mit $R^5$, $R^6$ = H mit einer Verbindung Xa oder

Xb, worin $L^4$ = eine Abgangsgruppe wie Halogen bedeutet,

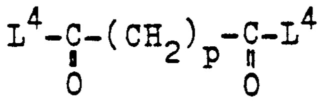

$L^4-CH_2-D-CH_2-L^4$

Xa Xb

umsetzt, oder

d) für R = 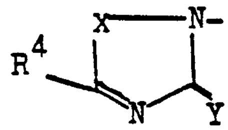

d1) für X = O eine Verbindung der Formel XI mit

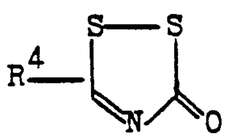 (XI) HONH-E (XII)

einer Verbindung der Formel XII umsetzt und die erhaltene Verbindung der Formel I mit Y = O auf bekannte Weise thioliert, oder

d2) für X = S eine gemäß a) erhaltene Verbindung der Formel I mit Y' = S und $R^5$, $R^6$ = H cyclisiert, oder

d) für R = 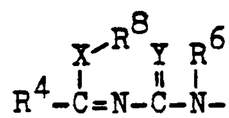

e1) eine Verbindung der Formel XIII

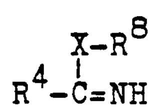

(XIII)

mit einer Verbindung der Formel VIa oder mit einer Verbindung der Formel

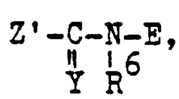

worin Z' die Bedeutung von Z außer $-NHR^5$ besitzt,
umsetzt, oder
e2) eine Verbindung der Formel XIV

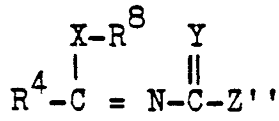 (XIV),

worin Z" die Bedeutung von Z' besitzt oder zusätzlich für Halogen steht, mit einer Verbindung der Formel III umsetzt, oder

f) für R = 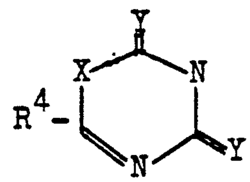

eine Verbindung der Formel II mit einer Verbindung der Formel VIa umsetzt, oder

g) für R = 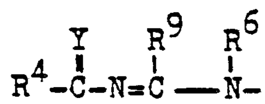

g1) eine Verbindung der Formel XV

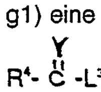 (XV)

mit einer Verbindung der Formel XVI

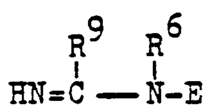 (XVI) umsetzt, oder

g2) eine Verbindung der Formel XVII

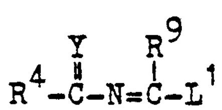 (XVII)

mit einer Verbindung der Formel III umsetzt, oder

g3) für $R^9$ = ein obengenannter Rest außer $-X-R^8$ eine unter a) erhaltene Verbindung der Formel (I) mit $Y^2$ = S und $R^5$, $R^6$ = H mit einer Verbindung der Formel $R^{9'}-SO-R^{9'}$ wobei $R^{9'}$ die Bedeutung von $R^9$ außer $-XR^8$ und $-S-S-R^{10}$ besitzt, oder mit einer Verbindung der Formel $L^5-S-S-R^{10}$, wobei $L^5$ = Halogen, N-Succinyl oder ein analoger Rest bedeutet, umsetzt, oder

h) für R = 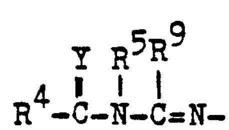

eine Verbindung der Formel XV mit einer Verbindung der Formel XVIII

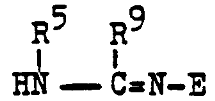 (XVIII)

umsetzt und die erhaltene Verbindung gegebenenfalls in ihr Salz überführt.

2. Verfahren gemäß Anspruch 1, dadurch gekennzeichnet, daß

A = N

R = einen Rest der Formeln

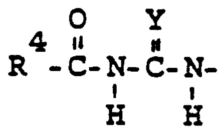 , 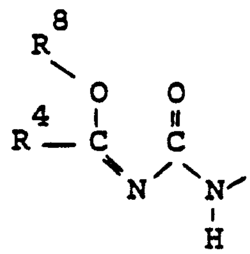 ,

oder 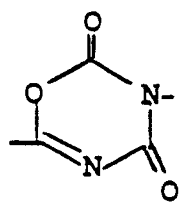

$R^1$ jeweils unabhängig voneinander Halogen, $(C_1-C_6)$-Alkyl oder $(C_1-C_6)$Alkoxy-carbonyl, wobei die Reste ein- oder mehrfach durch Halogen substituiert sein können,

$R^2$ jeweils unabhängig voneinander halogeniertes $(C_1-C_6)$-Alkyl, halogeniertes $(C_2-C_6)$-Alkenyl oder halogeniertes $(C_2-C_6)$-Alkinyl,

$R^3$ unabhängig voneinander Halogen,

$R^4$ Phenyl, das durch Halogen, $(C_1-C_3)$-Alkyl, $(C_1-C_3)$-Alkoxy, Halo$(C_1-C_3)$-alkyl oder Halo$(C_1-C_3)$-alkoxy substituiert sein kann,

$R^8$ $(C_1-C_6)$Alkyl, das halogeniert sein kann,

X unabhängig voneinander O oder S,

n im Falle des Phenoxyrestes 0 oder 1 und im Falle des Restes $R^3$ 0, 1 oder 2 und

m 0, 1, 2 oder 3 bedeuten.

3. Verfahren gemäß Anspruchen 2 oder 3, dadurch gekennzeichnet, daß

$R^1$ Halogen in den Positionen 2, 3 oder 5 des Phenoxyringes relativ zur Stellung von R, Halo $(C_1-C_6)$alkyl oder $(C_1-C_3)$Alkoxycarbonyl jeweils in den Positionen 3 oder 5 des Phenoxyringes

$R^2$ $(C_1-C_3)$-Haloalkoxy

$R^3$ F, Cl oder Br und

$R^4$ Phenyl bedeutet, das durch Halogen, insbesondere in den Positionen 2 oder 6 substituiert ist,

und A, $R^8$, X, n und m die in Anspruch 2 angegebenen Bedeutungen besitzen.

4. Verfahren zur Bekämpfung von Schadinsekten, Akariden, Nematoden und Mollusken, dadurch gekennzeichnet, daß man auf diese oder die von Ihnen befallenen Pflanzen, Flächen oder Substrate eine wirksame Menge einer Verbindung der Formel I appliziert.